# EUROPEAN PATENT APPLICATION

(11) **EP 4 679 070 A1**
(43) Date of publication of application: **14.01.2026**
(21) Application number: 25186042.5
(22) Date of filing: 27.06.2025
(51) Int. Cl.: G01N 27/02, G01N 27/22, G01N 27/12, H01M 10/42, G01N 33/00

(54) **SYSTEMS, APPARATUSES, AND METHODS FOR DETERMINING QUANTITIES OF MULTIPLE VAPORS USING A SINGLE SENSOR**

(30) Priority: 12.07.2024 US 202418771549
(71) Applicant: Honeywell International Inc., Charlotte, NC 28202 (US)
(72) Inventor: CHAPPLES, John, Charlotte, 28202 (US); PRATT, Keith Francis Edwin, Charlotte, 28202 (US); FOLEY, Thomas Mark, Charlotte, 28202 (US)
(74) Representative: Haseltine Lake Kempner LLP

(57) **Abstract**

Embodiments of the present disclosure provide systems, apparatuses, and methods for determining quantities of multiple vapors using a single sensor. In one embodiment, a method includes determining, by one or more processors an impedance of a sensing element; determining, by the one or more processors, a capacitance of the sensing element; and determining, by the one or more processors and based at least in part on (i) the impedance of the sensing element and (ii) the capacitance of the sensing element, (a) a quantity of a first vapor that has reacted with the sensing element and (b) a quantity of a second vapor that has reacted with the sensing element.

## Description

### TECHNICAL FIELD

Embodiments of the present disclosure generally relate to the field of vapor sensing, and specifically to systems, apparatuses, and methods for determining quantities of multiple vapors using a single sensor.

### BACKGROUND

Applicant has identified many technical challenges and difficulties associated with systems, apparatuses, and methods for determining quantities of multiple vapors using a single sensor. Through applied effort, ingenuity, and innovation, Applicant has solved problems related to systems, apparatuses, and methods for determining quantities of multiple vapors using a single sensor by developing solutions embodied in the present disclosure, which are described in detail below.

### BRIEF SUMMARY

In accordance with a first aspect of the disclosure, a method is provided. In some embodiments, the method is executable by one or more computing devices embodied in hardware, software, firmware, and/or any combination thereof as described herein. In some examples, the method may include determining, by one or more processors, an impedance of a sensing element; determining, by the one or more processors, a capacitance of the sensing element; and determining, by the one or more processors and based at least in part on (i) the impedance of the sensing element and (ii) the capacitance of the sensing element, (a) a quantity of a first vapor that has reacted with the sensing element and (b) a quantity of a second vapor that has reacted with the sensing element.

In some examples, the first vapor comprises an electrolyte vapor and the second vapor comprises a water vapor. In some examples, the sensing element comprises a polymer material and an ionic salt material. In some examples, the method includes causing transmission of, by the one or more processors and to the sensing element, a sinusoidal excitation signal having a frequency that is based at least in part on one or more properties of the sensing element, wherein determining the impedance of the sensing element is based at least in part on the sinusoidal excitation signal.

In some examples, determining (a) the quantity of the first vapor and (b) the quantity of the second vapor comprises: solving, by the one or more processors, (i) a first equation that specifies a relationship between the impedance of the sensing element, the quantity of the first vapor, and the quantity of the second vapor, and (ii) a second equation that specifies a relationship between the capacitance of the sensing element, the quantity of the first vapor, and the quantity of the second vapor. In some examples, the method further comprises determining, by the one or more processors, that the quantity of the first vapor satisfies a threshold quantity; and causing, by the one or more processors, a message to be displayed via a user interface, wherein the message alerts a user that the quantity of the first vapor satisfies the threshold quantity.

In some examples, the method further includes causing, by the one or more processors, a safety protocol to be performed based at least in part on the quantity of the first vapor. In some examples, the safety protocol comprises one or more of (i) venting a battery or (ii) disconnecting the battery. In some examples, the quantity of the first vapor is determined based at least in part on a sinusoidal excitation signal applied to the sensing element. In some examples, the method further comprises causing transmission of, by the one or more processors and to the sensing element, an excitation pulse; determining a second quantity of the first vapor based at least in part on the excitation pulse; and determining an error value associated with the second quantity of the first vapor based at least in part on a comparison of the second quantity of the first vapor and the quantity of the first vapor.

In accordance with a second aspect of the disclosure, an apparatus is provided. In one example embodiment of the apparatus, the apparatus includes one or more processors and a memory storing instructions that, when executed by the one or more processors, cause the apparatus to perform any one or more of the methods described herein. A second example apparatus includes means for performing each step of any one of the methods described herein.

In accordance with a third aspect of the disclosure, a system is provided. In one example embodiment of the system, the system includes a user interface and one or more processors in communication with the user interface, wherein the one or more processors are configured to perform any one or more of the methods described herein. In one example embodiment of the system, an example system includes at least one non-transitory computer-readable storage medium having computer program code stored thereon that, in combination with one or more processors, is configured for performing any one of the example methods described herein.

### BRIEF DESCRIPTION OF THE DRAWINGS

FIG. 1 illustrates an example system that supports determining quantities of multiple vapors using a single sensor.
FIG. 2 illustrates a cross-sectional view of an example sensor that supports determining quantities of multiple vapors using a single sensor.
FIG. 3 illustrates an example front view of an example polymer support that supports determining quantities of multiple vapors using a single sensor.
FIG. 4 illustrates an example front view of an example polymer support that supports determining quantities of multiple vapors using a single sensor.
FIG. 5 illustrates an overview of example batteries of the example system that support determining quantities of multiple vapors using a single sensor.
FIG. 6 illustrates an overview of an example battery pack of the example system that supports determining quantities of multiple vapors using a single sensor.
FIG. 7 illustrates an overview of an example battery pack of the example system that supports determining quantities of multiple vapors using a single sensor.
FIG. 8 illustrates an overview of an example battery pack of the example system that supports determining quantities of multiple vapors using a single sensor.
FIG. 9 illustrates an example impedance graph that supports determining quantities of multiple vapors using a single sensor.
FIG. 10 illustrates an example phase graph that supports determining quantities of multiple vapors using a single sensor.
FIG. 11 illustrates an example dataflow diagram that supports determining quantities of multiple vapors using a single sensor.
FIG. 12 illustrates an example computing device that supports determining quantities of multiple vapors using a single sensor.
FIG. 13 illustrates an example process that supports determining quantities of multiple vapors using a single sensor.

### DETAILED DESCRIPTION

Various embodiments of the present disclosure are described more fully hereinafter with reference to the accompanying drawings, in which some, but not all embodiments of the present disclosure are shown. Indeed, the present disclosure may be embodied in many different forms and should not be construed as limited to the embodiments set forth herein; rather, these embodiments are provided so that this disclosure will satisfy applicable legal requirements. The term "or" is used herein in both the alternative and conjunctive sense, unless otherwise indicated. The terms "illustrative" and "example" are used to be examples with no indication of quality level. Terms such as "computing," "determining," "generating," and/or similar words are used herein interchangeably to refer to the creation, modification, or identification of data. Further, "based on," "based at least in part on," "based at least on," "based upon," and/or similar words are used herein interchangeably in an open-ended manner such that they do not necessarily indicate being based only on or based solely on the referenced element or elements unless so indicated. Like numbers refer to like elements throughout.

### OVERVIEW

In many applications, it is often necessary to detect a vapor. For example, it may be necessary to detect a vapor associated with a battery (e.g., an electrolyte vapor that may be released from a battery) to detect if the battery is suffering from a fault and/or is overheating (e.g., due to a thermal runaway). In this regard, due to a variety of catalysts, such as poor design, overcharging, over-discharging, physical damage, and/or exposure to extreme temperatures (e.g., temperatures that cause the battery to overheat), batteries are susceptible to malfunctioning and/or thermal runaway. In a battery fault and/or a thermal runaway, the temperature of a battery may increase to a point that causes a chemical reaction to occur inside the battery, which further increases the temperature of the battery and causes more chemical reactions to occur. A battery fault and/or a thermal runaway may cause the battery to suffer a catastrophic failure, such as catching on fire, which may destroy the battery and also cause damage to nearby objects and/or individuals.

The resulting catastrophic failure of a battery, due to battery faults and/or thermal runaway, is particularly problematic in devices requiring large amounts of power, such as electric vehicles, because these devices may include hundreds of batteries (e.g., the hundreds of batteries may be organized into multiple battery packs with each battery pack including several batteries). As a result, if one battery suffers a catastrophic failure, this may cause other batteries in the battery pack and/or batteries in other battery packs to suffer a catastrophic failure (e.g., the catastrophic failure of one battery in an electric vehicle may cause all of the batteries in an electric vehicle to catch on fire and suffer a catastrophic failure). In such examples, catastrophic battery failures may result in the destruction of the electric vehicle, nearby objects (e.g., a garage the electric vehicle is parked in), and/or harm to nearby individuals.

In order to mitigate the risk of battery faults and/or thermal runaway, many batteries include a vent through which heat and vapors (e.g., vapors generated from chemical reactions in the battery) may be released. However, although a battery vent may allow some heat and vapors to escape from the battery, in many cases the vent alone is not capable of preventing a battery having a battery fault and/or in thermal runaway from suffering a catastrophic failure. As such, corrective action (e.g., stopping the charging of the battery) must be made by a user associated with the battery and/or other systems associated with the battery (e.g., a computing device associated with the battery). Accordingly, detecting a vapor and/or a specific quantity of the vapor may enable a user associated with the battery and/or other systems associated with the battery to take corrective action to remedy a fault and/or a thermal runaway condition to prevent the occurrence of a catastrophic failure.

Example solutions for detecting a vapor include sensors not specifically designed to detect vapors, such as, for example, a metal oxide sensor. However, metal oxide sensors have several drawbacks. For example, metal oxide sensors are generally non-specific sensors and, as a result, respond to any vapor that can modify the surface oxidation of the metal oxide sensor (e.g., including vapors that are not desired to be detected) and/or have low sensitivity (e.g., a metal oxide sensor can only detect vapors when there is a high concentration of the vapor near the sensor). As another example, metal oxide sensors use a moving average baseline to detect vapors and, as a result, metal oxide sensors are unable to detect the slow buildup of a vapor. As another example, metal oxide sensors are prone to baseline drift over time which decreases the accuracy of the metal oxide sensors over time. As another example, metal oxide sensors may be large in size and, as a result may be difficult to place near batteries (e.g., it may be difficult to place metal oxide sensors near a battery when the battery is in a confined space, such as in an electric vehicle). As another example, metal oxide sensors may be expensive to produce. As another example, metal oxide sensors may consume a large amount of power when operating (e.g., more than 10 milliwatts (mW) because metal oxide sensors typically have to be heated to high temperatures (e.g., greater than 200°C) to operate) and, as a result, it may be difficult to provide enough power such that a desired number of metal oxide sensors can be used in an application having many batteries (e.g., in an application having hundreds of batteries, it may be desirable to use multiple metal oxide sensors, but, due to the power consumption of metal oxide sensors, it may only be possible to provide enough power to use a less than desired number of metal oxide sensors).

Accordingly, systems that use metal oxide sensors to detect a vapor often have high rates of false alarms (e.g., due to metal oxide sensors being non-specific sensors), become inaccurate over time (e.g., due to metal oxide sensors being prone to baseline drift), are unable to detect the slow buildup of a vapor (e.g., due to metal oxide sensors using a moving average baseline to detect vapors), and may be difficult and/or expensive to implement in many applications (e.g., due to metal oxide sensors being large in size, being expensive to produce, and consuming large amounts of power when operating). These example drawbacks of metal oxide sensors decrease the usefulness of metal oxide sensors. Accordingly, there is a need for systems, apparatuses, and methods, that are capable of accurately and efficiently detecting a vapor, such as systems, apparatuses, and methods, that are capable of accurately and efficiently detecting an electrolyte vapor released from a battery.

In addition to the aforementioned challenges, various conventional techniques for detecting vapors may be incapable of differentiating between multiple types of vapors and/or determining respective quantities of vapors when a sensor is placed in an environment where multiple types of vapors are present. For example, a property of a metal oxide sensor, such as an impedance of the metal oxide sensor may be influenced by both water vapor and electrolyte vapor. Accordingly, in various sensing applications where both water vapor and electrolyte vapor are present (e.g., automotive battery sensing environments), existing sensing techniques may be incapable of discriminating between the effects of water vapor and the effects of electrolyte vapor. In such examples, the detection of water vapor quantities using dedicated humidity sensors may be intractable due to associated costs, power requirements, system complexity, and various design constraints (e.g., some battery compartments may not have space for multiple types of sensors). Accordingly, there is a need for systems, apparatuses, and methods, that are capable of accurately and efficiently detecting respective quantities of different vapors that may be present in a single sensing environment and/or accurately determining electrolyte vapor quantities in sensing environments where water vapor is present (e.g., reducing or eliminating false positives or inaccurate readings that may otherwise be triggered by water vapor).

Thus, to address these and/or other issues related to detecting one or more vapors, example systems, apparatuses, and methods are disclosed herein. For example, an embodiment in this disclosure, described in greater detail below, includes a sensor and/or associated processing techniques that can detect respective quantities of different types of vapors (e.g., water vapor and electrolyte vapor). In some examples, the sensor may include a substrate, a pair of electrodes disposed on the substrate, and a polymer support disposed on the substrate such that the polymer support is in contact with the pair of electrodes. In some examples, the polymer support may include an ionic salt. In some examples, the polymer support may be configured to absorb at least some of one or more vapors and, when the polymer support absorbs at least some of the one or more vapors, one or more properties of the polymer support, such as a conductivity of the polymer support may change (e.g., the conductivity may increase). In some examples, the impedance of the polymer support and/or a phase angle associated with the polymer support may be measured or otherwise determined. A quantity of a first vapor of the one or more vapors and/or a quantity of a second vapor of the one or more vapors may then be determined based on the impedance and/or the phase angle (e.g., by simultaneously solving two equations that correlate impedance and/or phase angle values to quantities of the first vapor and/or the second vapor). Accordingly, in some examples, the sensor may be able to accurately detect (e.g., without a high risk of false alarms) whether a battery fault and/or thermal runaway is occurring (e.g., based on a presence and/or quantity of electrolyte vapor). Thus, the described systems, apparatuses, and methods may enable a user and/or computing device associated with the battery to take corrective action to prevent a catastrophic failure.

### DEFINITIONS

In some embodiments, the term "one or more processors" refers to one or more components or devices that are configurable to perform one or more operations, calculations, determinations, communications, or logical processes. In some examples, a processor may be a subcomponent of a computing device. In some other examples, however, one or more processors may be implemented as virtualized elements of a virtualized computing system or architecture. In some examples, the one or more processors may be included in a sensor device and/or sensor system. For example, a gas sensing device may include one or more processors and/or one or more sensing elements in communication with the one or more processors. The one or more processors may be configured to perform any one or more of the operations described herein.

In some embodiments, the term "sensing element" refers to a component configured to sense one or more phenomena. For example, a sensing element may exhibit a response to a phenomenon, which may be measured and/or quantified. In some examples, a sensing element may be a composite material or structure. For example, a sensing element may include a polymer (e.g., a polymer film) and an ionic salt that is embedded in or layered onto the polymer film. In some examples, one or more properties and/or characteristics of a sensing element may change in response to one or more phenomena and/or stimuli. For example, a conductivity of a sensing element may increase or decrease when one or more vapors react with the sensing element. Additionally, or alternatively, a capacitance of the sensing element may increase or decrease when one or more vapors react with the sensing element. As described herein, the terms "sensing element" and "sensor" may be used interchangeably without loss of meaning.

In some examples, an excitation signal and/or an excitation pulse (e.g., an electrical signal and/or pulse) may be transmitted towards and/or through a sensing element. For example, a first and second electrode (e.g., conductor) may be coupled with the sensing element. In such examples, an excitation signal and/or an excitation pulse may be routed through the sensing element via the first electrode and the second electrode (e.g., the electrical signal may enter the sensing element through the first electrode and exit the sensing element through the second electrode). In some examples, one or more characteristics of an excitation signal may be selected (e.g., by a user, by a computing device) based on one or more properties of the sensing element. For example, a frequency of an excitation signal (e.g., an alternating current (AC) electrical signal) may be selected based on a quantity of an ionic salt material in the sensing element. In such examples, the one or more characteristics of the excitation signal may be selected to elicit a specific or known response by the sensing element.

In some embodiments, the term "impedance" refers to a measure or value representative of the opposition to alternating current. For example, an electric circuit and/or component may exhibit or otherwise be associated with an impedance. In some examples, impedance may arise from the combined effect of ohmic resistance and reactance. In one example of a circuit element coupled with two terminals (e.g., a sensing element), an impedance value may be calculated as a ratio of a complex representation of a sinusoidal voltage between the terminals to a complex representation of the current flowing through the circuit element.

As described herein, an impedance value (e.g., of a sensing element) may be determined by one or more processors. In such examples, the one or more processors may cause an excitation signal (e.g., a sinusoidal signal, a pulse) to be applied to a sensing element (e.g., by one or more signal generation components). For example, the one or more processors may transmit one or more control signals to one or more signal generation components, which may generate and/or transmit one or more excitation signals to the sensing element in response to receiving the one or more control signals. In some examples, the one or more processors may then measure the impedance or otherwise cause the impedance to be measured (e.g., by comparing the excitation signal to a signal output by the sensing element).

In some examples, an impedance value of a sensing element may be indicative of or representative of a quantity of a vapor that has reacted with or otherwise been exposed to the sensing element. For example, an electrolyte vapor may react with one or more materials of a sensing element and cause an impedance of the sensing element to decrease. Additionally, or alternatively, a conductivity of the sensing element may rise in response to the electrolyte vapor reacting with the one or more materials of the sensing element. In some examples, water vapor may additionally, or alternatively cause an impedance of the sensing element to decrease. However, the sensing element may be less sensitive to water vapor than electrolyte vapor. Accordingly, the impedance of the sensing element may decrease more in the presence of electrolyte vapor than in the presence of water vapor.

In some embodiments, the term "capacitance" refers to a value representative of an object, material, and/or electrical component's ability to store electric charge. For example, a component of an electrical circuit, such as a sensing element, may exhibit or otherwise be associated with a capacitance. As described herein, a capacitance value (e.g., of a sensing element) may be determined by one or more processors. In such examples, the one or more processors may cause an excitation signal (e.g., a sinusoidal signal, a pulse) to be applied to a sensing element (e.g., by one or more signal generation components). For example, the one or more processors may transmit one or more control signals to one or more signal generation components, which may generate and/or transmit one or more excitation signals to the sensing element in response to receiving the one or more control signals. In some examples, the one or more processors may then measure the capacitance or otherwise cause the capacitance to be measured (e.g., by comparing the excitation signal to a signal output by the sensing element). In some examples, a phase angle associated with the excitation signal may be indicative of a capacitance of the sensing element. For example, a phase angle of an impedance measurement may be indicative of a capacitance of the sensing element (e.g., a phase angle between an input excitation signal and an output signal output by a sensing element).

In some examples, a capacitance value of a sensing element may be indicative of or representative of a quantity of a vapor that has reacted with or otherwise been exposed to the sensing element. For example, water vapor may react with one or more materials of a sensing element and cause a capacitance of the sensing element to increase or decrease. In some examples, an electrolyte vapor may additionally, or alternatively cause a capacitance of the sensing element to increase or decrease. However, the sensing element may be less sensitive to the electrolyte vapor than water vapor. Accordingly, the capacitance of the sensing element may increase or decrease more in the presence of water vapor than in the presence of the electrolyte vapor.

In some embodiments, the term "vapor" refers to a substance or phase of matter, which may be airborne or otherwise suspended. A vapor may be a substance that is in a gaseous state. In some examples, a vapor may react with one or more other substances or materials. For example, a sensing element (e.g., one or more materials of a sensing element) may absorb one or more vapors. In some examples, a vapor may be emitted by a device or substance. For example, a battery may emit an electrolyte vapor (e.g., a first vapor) in a specific scenario or context. In some examples, a battery may emit an electrolyte vapor in one or more failure modes (e.g., if thermal runaway is occurring at the battery). In such examples, the presence of an electrolyte vapor and/or a specific quantity of an electrolyte vapor (e.g., a threshold quantity) may be indicative of a battery experiencing thermal runaway. In some examples, the term "vapor" may be utilized herein to refer to a substance in a gaseous phase, although the substance may commonly (e.g., at room temperature) exist in one or more other phases. For example, the term "vapor" may refer to water vapor, which may commonly be observed in a liquid phase and/or a solid phase. In some examples, the term "vapor" may be utilized herein to refer to any gas and/or substance that commonly occurs a gaseous phase. For example, the term "vapor" may refer to oxygen, which more commonly (e.g., at room temperature) exists in the gaseous phase.

As described herein, an electrolyte vapor may include, refer to, or otherwise be an example of one or more types of electrolyte vapors. For example, some non-limiting examples of electrolyte vapors may include propylene carbonate (PC), ethylene carbonate (EC), diethyl carbonate (DEC), dimethyl carbonate (DMC), ethyl methyl carbonate (EMC), dimethoxyethane (DME), gamma-butyrolactone (GBL), and/or the like.

In some examples, a sensing element may be exposed to and/or react with two or more vapors (e.g., simultaneously). For example, a sensing element may react with an electrolyte vapor (e.g., a first vapor) and water vapor (e.g., humidity, a second vapor). In such examples, the techniques described herein may enable one or more processors to determine quantities of both vapors that have reacted with the sensing element.

In some embodiments, the term "quantity" refers to an amount, volume, or concentration of a substance, such as a vapor. A quantity may be expressed as a value with reference to one or more units of measure. For example, a quantity of a vapor may be expressed as a quantity of milliliters (mLs), moles (mols), and/or the like. In some examples, one or more properties or characteristics of a sensing element may be indicative of a quantity of one or more vapors that have reacted with the sensing element. For example, an impedance of a sensing element may be indicative of a quantity of water vapor and/or a quantity of electrolyte vapor that have reacted with the sensing element.

In some embodiments, the term "threshold quantity" refers to a value, level, or limit that serves as a reference or trigger for performing one or more actions and/or operations. A threshold quantity may be an upper limit or a lower limit. In some examples, a threshold quantity may be satisfied when a value and/or a quantity is greater than, less than, or equal to the threshold quantity. As described herein, one or more processors may determine if a quantity of a vapor satisfies a threshold quantity (e.g., a threshold vapor quantity). In such examples, the threshold quantity may be a quantity of a vapor (e.g., an electrolyte vapor) that is indicative of a failure mode of a battery or a quantity of vapor that otherwise presents one or more hazards. In some examples, the threshold quantity may be satisfied if a measured quantity of the vapor is greater than or equal to the threshold quantity.

In some embodiments, the term "react" refers to a process or interaction between any combination of two or more substances, materials, or compounds. For example, a vapor that comes into contact with a sensing element may cause one or more reactions to occur (e.g., an ionic salt of the sensing element may solvate). Accordingly, the vapor may be said to react with the sensing element. In some examples, a reaction may include one or more absorption processes. For example, a reaction may include a sensing element absorbing one or more vapors.

In some embodiments, the term "excitation signal" refers to an electrical signal that may be used to determine one or more characteristics of a sensing element. For example, an impedance and/or a capacitance of a sensing element may be determined based on an excitation signal. An excitation signal may be a digital or analog signal. In some examples, an excitation signal may be a waveform, such as a sinusoidal excitation signal, a sawtooth excitation signal, a square wave excitation signal, and/or the like. In some examples, an excitation signal may include one or more pulses. In some examples, an excitation signal may be generated by one or more processors or a signal generation component that is controlled by the one or more processors. In some examples, an excitation signal may have a frequency, which may be selected by a user or the one or more processors to achieve a specific goal. For example, a frequency of an excitation signal may selected based on test results that indicate an optimal frequency for the excitation signal (e.g., a frequency that results in a more pronounced response by a sensing element when compared to one or more other frequencies that are tested).

In some examples, an excitation signal may be transmitted to and/or across a sensing element. In such examples, a response of the sensing element may be measured, which may be indicative of a quantity of one or more vapors that have reacted with the sensing element. For example, the response of the sensing element may indicate an impedance of the sensing element and/or a phase angle of the sensing element (e.g., a capacitance of the sensing element).

Although some examples described herein refer to measuring impedance and/or capacitance of a sensing element using an excitation signal with a specific frequency, one or more variable frequency approaches may be utilized. For example, a spectroscopy approach may be utilized in which a plurality of excitation signals having a plurality of frequencies are transmitted to a sensing element. In some examples, a frequency scanning operation may be utilized to modulate an excitation signal (e.g., scan through a plurality of frequencies), which may enable one or more quantities of one or more vapors to be determined based on a frequency response of the sensing element to the modulated excitation signal.

In some embodiments, the term "pulse" refers to a type of electrical signal, such as an excitation signal (e.g., an excitation pulse). For example, a pulse may be a first type of excitation signal and a sinusoidal excitation signal may be a second type of excitation signal. In some examples, a pulse may be a type of direct current (DC) signal and a sinusoidal signal may be a type of AC signal. In some examples, a waveform may include one or more pulses, which may each have pulse widths.

In some examples, a pulse may be utilized to determine one or more characteristics of a sensing element. For example, one or more processors may cause the transmission of a pulse to a sensing element. An impedance, capacitance, and/or time decay may then be determined based on the pulse. As described herein, a sinusoidal excitation signal may additionally, or alternatively be utilized to determine the one or more characteristics of the sensing element. However, generating a sinusoidal excitation signal (or any other type of AC excitation signal) may be more costly than generating a pulse excitation signal. For example, one or more hardware components for generating a sinusoidal excitation signal may be more costly than one or more hardware components for generating a pulse excitation signal. Additionally, or alternatively, various signal processing operations may be more resource intensive for sinusoidal excitation signals when compared to pulse excitation signals.

Accordingly, in one example embodiment of the present disclosure, one or more pulse excitation signals (e.g., one or more excitation pulses) may be utilized to determine one or more vapor quantities during a first time period and one or more sinusoidal excitation signals may be utilized to determine one or more vapor quantities during a second time period that is shorter than the first time period. Such techniques may enable the selective utilization of more computationally intensive sensing operations (e.g., using sinusoidal excitation signals) to achieve more accurate vapor sensing results that would not otherwise be achievable with less computationally intensive sensing operations.

In some examples, a vapor sensing operation that utilizes an AC excitation signal may be performed if a quantity of a specific vapor is determined to have satisfied a threshold quantity based on a vapor sensing operation that utilizes a DC excitation signal (e.g., an excitation pulse). For example, a first type of vapor sensing operation (e.g., that utilizes a DC excitation pulse, a lower cost sensing operation) may be performed periodically and/or continuously. If a quantity of a vapor (e.g., an electrolyte vapor) that is detected using the first type of vapor sensing operation satisfies a threshold quantity, a second type of vapor sensing operation may be triggered (e.g., as a verification step, to determine an error value and/or validity of the previously detected vapor quantity). The second type of vapor sensing operation may be more costly than the first type of vapor sensing operation. However, the second type of vapor sensing operation (e.g., a vapor sensing operation using a sinusoidal excitation signal and/or a spectroscopy-based vapor sensing operation) may be more accurate than the first type of vapor sensing operation. For example, the second type of vapor sensing operation may be capable of correcting error in the first type of vapor sensing operation associated with the presence of humidity, which may result in improved accuracy of electrolyte vapor measurements.

In some embodiments, the term "equation" refers to a statement that the values of two mathematical expressions are equal. As described herein, a first equation may relate a first characteristic (e.g., an impedance) of a sensing element to a quantity of a first vapor (e.g., an electrolyte vapor) that has reacted with the sensing element and a quantity of a second vapor (e.g., water vapor) that has reacted with the sensing element. A second equation may relate a second characteristic (e.g., a phase angle of the impedance, a capacitance) of the sensing element to the quantity of the first vapor that has reacted with the sensing element and the quantity of the second vapor that has reacted with the sensing element. In some examples, the first characteristic and the second characteristic may be determined or otherwise measured (e.g., by one or more processors). The one or more processors may then perform one or more operations and/or calculations to determine the quantity of the first vapor and the quantity of the second vapor based on the first characteristic and the second characteristic. For example, the one or more processors may solve (e.g., algebraically) the system of equations including the first equation and the second equation.

In some embodiments, the term "safety protocol" refers to a procedure or operation that is performed to achieve one or more safety outcomes. For example, one or more processors may initiate or otherwise cause one or more safety protocols to be performed. A safety protocol may include deactivating, deenergizing, and/or disconnecting one or more electrical components. For example, a safety protocol may include disconnecting a battery from one or more devices (e.g., a battery in an electric vehicle may be disconnected from an electric motor or any other electrical component that draws current from the battery). In some examples, a safety protocol may include performing one or more operations to vent one or more vapors from a battery and/or a compartment that stores a battery. For example, one or more processors may send a control signal to an electronically actuated venting component that vents a battery in response to a determination that an electrolyte vapor quantity satisfies a threshold quantity.

In some embodiments, the term "user interface" refers to hardware and/or software that is configured to interface with one or more individuals (e.g., one or more users). For example, a user interface may be a device that receives one or more inputs from a user and/or provides one or more outputs to the user, such as a monitor, a display, a speaker, a microphone, a printer, a keyboard, a mouse, a joystick, and/or the like. In some examples, a user interface may be a software application, such as a graphical user interface that is displayed and/or executed on a computing device. In some examples, a user interface may provide an audio and/or visual representation of information. For example, a user interface of a computing device, such as a smartphone, may display one or more images, which may be viewed and/or interacted with by one or more individuals (e.g., via a touchscreen, via one or more buttons). In some examples, an image displayed via a user interface may include a text string, which may be representative of a text string located on an object in a real-world environment (e.g., an image displayed on a user interface may include a representation of a text string located on a shipping container, a package, a product, a document (e.g., an identification document), and/or the like).

In some embodiments, the term "error value" refers to a value indicative of a degree of inaccuracy associated with a measurement. In some examples, an error value may indicate a difference between a ground-truth value and a measured value. In some examples, an error value may indicate a difference between a value that is measured or otherwise determined using a first vapor sensing operation and a value that is measured or otherwise determined using a second vapor sensing operation. For example, an error value may be indicative of a difference between a vapor quantity that is determined using an AC excitation signal and a vapor quantity that is determined using a DC excitation signal.

### EXAMPLE SYSTEMS AND PROCESSES OF THE DISCLOSURE

Embodiments of the present disclosure may be implemented in various ways, including as computer program products that comprise articles of manufacture. Such computer program products may include one or more software components including, for example, software objects, methods, data structures, or the like. A software component may be coded in any of a variety of programming languages. An illustrative programming language may be a lower-level programming language such as an assembly language associated with a particular hardware architecture and/or operating system platform. A software component comprising assembly language instructions may require conversion into executable machine code by an assembler prior to execution by the hardware architecture and/or platform. Another example programming language may be a higher-level programming language that may be portable across multiple architectures. A software component comprising higher-level programming language instructions may require conversion to an intermediate representation by an interpreter or a compiler prior to execution.

Other examples of programming languages include, but are not limited to, a macro language, a shell or command language, a job control language, a script language, a database query, or search language, and/or a report writing language. In one or more example embodiments, a software component comprising instructions in one of the foregoing examples of programming languages may be executed directly by an operating system or other software component without having to be first transformed into another form. A software component may be stored as a file or other data storage construct. Software components of a similar type or functionally related may be stored together, such as in a particular directory, folder, or library. Software components may be static (e.g., preestablished, or fixed) or dynamic (e.g., created or modified at the time of execution).

A computer program product may include a non-transitory computer-readable storage medium storing applications, programs, program modules, scripts, source code, program code, object code, byte code, compiled code, interpreted code, machine code, executable instructions, and/or the like (also referred to herein as executable instructions, instructions for execution, computer program products, program code, and/or similar terms used herein interchangeably). Such non-transitory computer-readable storage media include all computer-readable media (including volatile and non-volatile media).

In some embodiments, a non-volatile computer-readable storage medium may include a floppy disk, flexible disk, hard disk, solid-state storage (SSS) (e.g., a solid-state drive (SSD), solid state card (SSC), solid state module (SSM), enterprise flash drive, magnetic tape, or any other non-transitory magnetic medium, and/or the like). A non-volatile computer-readable storage medium may also include a punch card, paper tape, optical mark sheet (or any other physical medium with patterns of holes or other optically recognizable indicia), compact disc read only memory (CD-ROM), compact disc-rewritable (CD-RW), digital versatile disc (DVD), Blu-ray disc (BD), any other non-transitory optical medium, and/or the like. Such a non-volatile computer-readable storage medium may also include read-only memory (ROM), programmable read-only memory (PROM), erasable programmable read-only memory (EPROM), electrically erasable programmable read-only memory (EEPROM), flash memory (e.g., Serial, NAND, NOR, and/or the like), multimedia memory cards (MMC), secure digital (SD) memory cards, SmartMedia cards, CompactFlash (CF) cards, Memory Sticks, and/or the like. Further, a non-volatile computer-readable storage medium may also include conductive-bridging random access memory (CBRAM), phase-change random access memory (PRAM), ferroelectric random-access memory (FeRAM), non-volatile random-access memory (NVRAM), magnetoresistive random-access memory (MRAM), resistive random-access memory (RRAM), Silicon-Oxide-Nitride-Oxide-Silicon memory (SONOS), floating junction gate random access memory (FJG RAM), Millipede memory, racetrack memory, and/or the like.

In some embodiments, a volatile computer-readable storage medium may include random access memory (RAM), dynamic random access memory (DRAM), static random access memory (SRAM), fast page mode dynamic random access memory (FPM DRAM), extended data-out dynamic random access memory (EDO DRAM), synchronous dynamic random access memory (SDRAM), double data rate synchronous dynamic random access memory (DDR SDRAM), double data rate type two synchronous dynamic random access memory (DDR2 SDRAM), double data rate type three synchronous dynamic random access memory (DDR3 SDRAM), Rambus dynamic random access memory (RDRAM), Twin Transistor RAM (TTRAM), Thyristor RAM (T-RAM), Zero-capacitor (Z-RAM), Rambus in-line memory module (RIMM), dual in-line memory module (DIMM), single in-line memory module (SIMM), video random access memory (VRAM), cache memory (including various levels), flash memory, register memory, and/or the like. It will be appreciated that where embodiments are described to use a computer-readable storage medium, other types of computer-readable storage media may be substituted for, or used in addition to, the computer-readable storage media described above.

As should be appreciated, various embodiments of the present disclosure may also be implemented as methods, apparatuses, systems, computing devices, computing entities, and/or the like. As such, embodiments of the present disclosure may take the form of an apparatus, system, computing device, computing entity, and/or the like executing instructions stored on a computer-readable storage medium to perform certain steps or operations. Thus, embodiments of the present disclosure may also take the form of an entirely hardware embodiment, an entirely computer program product embodiment, and/or an embodiment that comprises a combination of computer program products and hardware performing certain steps or operations.

Embodiments of the present disclosure are described below with reference to block diagrams and flowchart illustrations. Thus, it should be understood that each block of the block diagrams and flowchart illustrations may be implemented in the form of a computer program product, an entirely hardware embodiment, a combination of hardware and computer program products, and/or apparatuses, systems, computing devices, computing entities, and/or the like carrying out instructions, operations, steps, and similar words used interchangeably (e.g., the executable instructions, instructions for execution, program code, and/or the like) on a computer-readable storage medium for execution. For example, retrieval, loading, and execution of code may be performed sequentially such that one instruction is retrieved, loaded, and executed at a time. In some example embodiments, retrieval, loading, and/or execution may be performed in parallel such that multiple instructions are retrieved, loaded, and/or executed together. Thus, such embodiments may produce specifically configured machines performing the steps or operations specified in the block diagrams and flowchart illustrations. Accordingly, the block diagrams and flowchart illustrations support various combinations of embodiments for performing the specified instructions, operations, or steps.

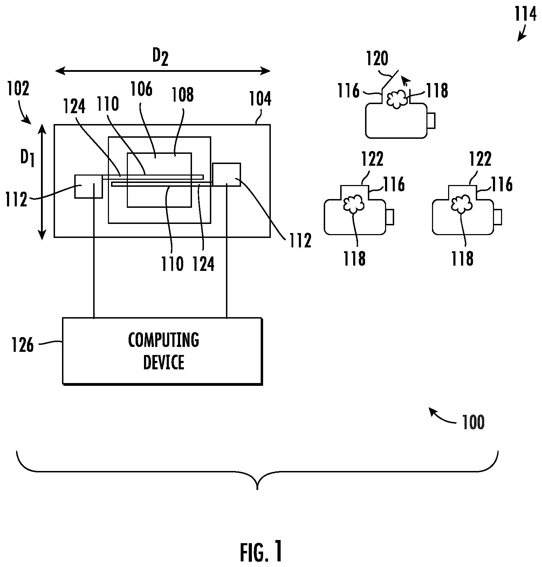

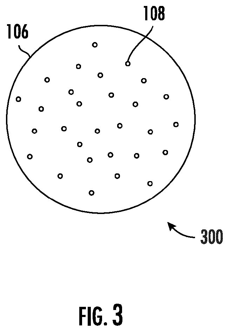

With reference to FIGS. 1-5 embodiments described herein include a system 100 for detecting a vapor 118. In some embodiments, the system 100 for detecting a vapor 118 may include a sensor 102. In some embodiments, the sensor 102 may be able to detect any vapor that may solvate an ionic salt. For example, the sensor 102 may be able to detect a vapor associated with a battery (e.g., an electrolyte vapor that may be released from a battery). In some embodiments, the sensor 102 may have dimensions D₁ and D₂. In some embodiments, D₁ may be less than 10 millimeters (mm) and/or D₂ may be less than 10mm. For example, in some embodiments, D₁ may be approximately 1mm and/or D₂ may be approximately 1 mm.

In some embodiments, the sensor 102 may include a substrate 104. The substrate 104 may comprise one or more of silicon, silicon oxide, silicon nitride, borosilicate glass, quartz, silica, sapphire, alumina, or plastic. Said differently, the substrate 104 may be comprised of any material capable of ensuring that the sensor 102 may detect a vapor 118. In this regard, for example, the substrate 104 may be a printed circuit board.

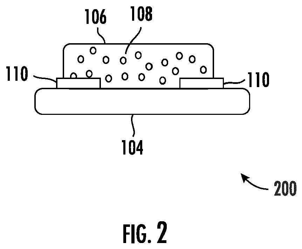

In some embodiments, the sensor 102 may include a pair of electrodes 110 disposed on the substrate 104. In some embodiments, the sensor 102 may include a polymer support 106 disposed on the substrate 104. The polymer support 106 may be disposed on the substrate 104 such that the polymer support 106 is in contact with each of the pair of electrodes 110. The pair of electrodes 110 may comprise one or more of copper, nickel, cobalt, tungsten, silicon carbide, palladium, platinum, gold, or transition metal alloys. For example, as depicted in FIGS. 1 and 2, each of the pair of electrodes 110 may be in contact with a portion of the polymer support 106. In this regard, for example, the polymer support 106 may be configured to provide a connection between the pair of electrodes 110 (e.g., the pair of electrodes 110 may not be in contact with each other). In some embodiments, the pair of electrodes 110 may be disposed on the substrate 104 in any configuration such that the impedance of the polymer support 106 and/or phase angle associated with the polymer support 106 may be measured. For example, each of the pair of electrodes 110 may be interdigitated electrodes. As another example, each of the pair of electrodes 110 may be disposed on the substrate 104 such that the pair of electrodes 110 form a spiral. In some embodiments, a distance between the pair of electrodes 110 may be approximately equal to a thickness of the polymer support 106.

In some embodiments, the polymer support 106 maybe any shape such that when disposed on the substrate 104, the polymer support 106 may be in contact with the pair of electrodes 110. For example, the polymer support 106 may be cylindrical, cubical, rectangular, and/or the like. In some embodiments, the polymer support 106 may be comprised of one or more thermoplastics (e.g., a non-reactive thermoplastic). For example, the polymer support 106 may comprise one or more of poly(ethyl methacrylate), poly(butyl methacrylate-co-methyl methacrylate), poly(methyl methacrylate-co-ethyl acrylate), poly(ethylene oxide) (PEO), poly(vinyl pyrrolidone) (PVP), poly(acrylonitrile) (PAN), poly(vinyl acetate) (PVAc), poly(vinyl butyral-co-vinyl alcohol-co-vinyl acetate), poly(ethylene-co-vinyl acetate), poly(1-vinylpyrrolidone-co-vinyl acetate), poly(methyl methacrylate) (e.g., PMMA), poly(vinylidene fluoride) (e.g., PVDF), poly(vinylidene fluoride-co-trifluoroethylene) (e.g., PVDF-TrFE), poly(vinylidene fluoride-co-hexafluoropropylene) (e.g., PVDF-HEP), poly(dimethyldiallylammonium) bis(fluorosulfonyl)imide (e.g., PDDA FSI), or poly(dimethylpyrrolidinium) bis(trifluoromethylsulfonyl)imide (e.g., PDP TFSI).

In some embodiments, the polymer support 106 may include an ionic salt 108. In some embodiments, the ionic salt 108 may be dispersed throughout the polymer support 106. For example, the ionic salt 108 may be dispersed in clumps throughout the polymer support 106. As another example, the ionic salt 108 may be dispersed throughout the polymer support 106 in a substantially uniform distribution. In some embodiments, the ionic salt 108, may be dispersed in clumps throughout the polymer support 106 and/or throughout the polymer support 106 in a substantially uniform distribution before the sensor 102 has detected a vapor 118.

In some embodiments, the ionic salt 108 may be comprised of one or more of tetrabutylammonium tetrafluoroborate, tetraethylammonium tetrafluoroborate, tetramethylammonium tetrafluoroborate, lithium tetrafluoroborate, silver tetrafluoroborate, tetramethylammonium hexafluorophosphate, tetraethylammonium hexafluorophosphate, tetrabutylammonium hexafluorophosphate, tetrabutylammonium chloride, tetraethylammonium chloride, tetramethylammonium chloride, tetramethylammonium bis(trifluoromethylsulfonyl)imide, tetrabutylammonium bis(trifluoromethylsulfonyl)imide, tributylmethylammonium bis(trifluoromethylsulfonyl)imide, tetraethylammonium bis(trifluoromethylsulfonyl)imide, tetrabutylammonium triflate, tributylmethylammonium triflate, 1-ethyl-3-methylimidazolium bis(trifluoromethylsulfonyl)imide, or triethyl sulfonium bis(trifluoromethyl sulfonyl)imide. In some embodiments, the ionic salt 108 may be chosen based on the ionic salt's solubility in the vapor 118, the ionic salt's ability to form an ionically conducting electrolyte after the ionic salt 108 has been exposed to the vapor 118, and/or the ionic salt's 108 compatibility with the polymer support 106.

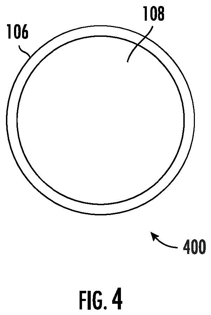

In some embodiments, the polymer support 106 may be configured to react with or otherwise absorb at least some of the vapor 118. For example, the polymer support 106 may absorb some of a vapor 118 when a vapor 118 is in the presence of the sensor 102. In some embodiments, absorbing at least some of a vapor 118 may cause the polymer support 106 to solvate. In this regard, for example, the polymer support 106 may become more flexible. In some embodiments, absorbing at least some of a vapor 118 may cause the ionic salt 108 to solvate. In this regard, such as depicted in FIG. 4 for example, the ionic salt 108 may dissolve into the polymer support 106 (e.g., the ionic salt 108 may dissociate into ions). In some embodiments, the polymer support 106 may absorb some of a vapor 118 (and solvate the polymer support 106 and/or the ionic salt 108) within one minute of the vapor 118 being in the presence of the sensor 102. In some embodiments, absorbing at least some of a vapor 118 and, as a result, causing the polymer support 106 and/or the ionic salt 108 to solvate, causes a conductivity of the polymer support 106 to increase. In this regard, for example, if the conductivity of the polymer support 106 increases, the impedance of the polymer support 106 may decrease. As another example, if the conductivity of the polymer support 106 increases, the phase angle associated with the polymer support 106 (e.g., the phase angle of the impedance of the polymer support 106) may shift (e.g., the phase angle associated with the polymer support 106 may shift such that the phase angle exceeds a phase angle threshold).

In some embodiments, the vapor 118 may be any vapor that may solvate the ionic salt 108. For example, as described above, the vapor 118 may be associated with one or more batteries 114. That is, in some embodiments, the vapor 118 may be released from one of the one or more batteries 114. In some embodiments, the vapor 118 may be comprised of one or more of propylene carbonate (PC), ethylene carbonate (EC), diethyl carbonate (DEC), dimethyl carbonate (DMC), ethyl methyl carbonate (EMC), dimethoxyethane (DME), or gamma-butyrolactone (GBL).

In some embodiments, the sensor 102 may include a pair of contact pads 112 disposed on the substrate 104. In some embodiments, each of the pair of contact pads 112 may be in communication (e.g., electrical communication) with one of the pair of electrodes 110. In some embodiments, each of the pair of contact pads 112 may be in communication (e.g., electrical communication) with one of the pair of electrodes 110 via an associated connection path 124. In some embodiments, each connection path 124 may comprise one or more electrical connection members, such as electrical wires, electrical leads, electrical traces, and/or the like.

In some embodiments, each of the pair of contact pads 112 may be configured to be connected to a computing device 126. In this regard, the sensor 102 may be configured to be connected to the computing device 126 via each of the pair of contact pads 112 such that the sensor 102 and the computing device 126 may be in communication (e.g., electrical communication). In some embodiments, the sensor 102 and the computing device 126 may be connected by one or more electrical connection members, such as electrical wires, electrical leads, electrical trades, and/or the like. Although the sensor 102 and the computing device 126 are depicted as separate components in FIG. 1, it would be understood by one skilled in the field to which this disclosure pertains that, in some embodiments, the sensor 102 and the computing device 126 may be combined into a single component. For example, the sensor 102 may integrated into the computing device 126 or the computing device 126 may be integrated into the sensor 102.

As described above, in some embodiments, the system 100 for detecting a vapor 118 may include one or more batteries 114. The one or more batteries 114 may be any kind of battery used in a variety of applications (e.g., in electric vehicles). For example, the one or more batteries 114 may include lithium-ion batteries, lithium-polymer batteries, alkaline batteries, nickel metal hydride batteries, carbon zinc batteries, silver oxide batteries, zinc air batteries, single use batteries, rechargeable batteries, and/or the like. In some embodiments, the one or more batteries 114 may include more than one type of battery. For example, one of the one or more batteries 114 may be a lithium-ion battery and another of the one or more batteries 114 may be a lithium-polymer battery.

In some embodiments, each of the one or more batteries 114 may include a vent 116. In some embodiments, the vent 116 may be located at any location on each of the one or more batteries 114 (e.g., on the top of each of the one or more batteries 114). The vent 116 of each of the one or more batteries 114 may be configured to move between a closed position 122 and an open position 120. In some embodiments, the vent 116 of each of the one or more batteries 114 may be in the closed position 122 when the battery is operating normally (e.g., the battery is undamaged and/or operating at a normal temperature). In some embodiments, the vent 116 of each of the one or more batteries 114 may be in the open position 120 when the battery is not operating normally (e.g., the battery is damaged and/or not operating at a normal temperature). For example, the vent 116 of one of the one or more batteries 114 may be in the open position 120 when a temperature of the battery is above a temperature threshold. In some embodiments, the vent 116 of one of the one or more batteries 114 may be configured to move from the closed position 122 to the open position 120 when the temperature of the battery is above the temperature threshold. In some embodiments, the temperature threshold may be based on the size (e.g., the size of a battery cell in the one or more batteries 114), material (e.g., the material of a battery cell in the one or more batteries 114), and/or design (e.g., the design of a battery cell in the one or more batteries 114) of the one or more batteries 114. In some embodiments, the temperature threshold may be between approximately 130°C and 200°C.

In some embodiments, the vent 116 of one or more of the one or more batteries 114 may be configured to move from the closed position 122 to the open position 120 to release a vapor 118 from the battery and, in some embodiments, heat from the battery. In some embodiments, the vent 116 of one of the one or more batteries 114 may be configured to move from the closed position 122 to the open position 120 to release a vapor from the battery when the battery is overheating (e.g., going into thermal runaway) and/or due to a fault associated with the battery (e.g., a fault not associated with the battery overheating). For example, as the temperature of one of the one or more batteries 114 increases, a vapor 118 may build up inside the battery. In some embodiments, once a certain amount of the vapor 118 has built up inside one of the one or more batteries 114 (e.g., when the temperature of the battery is above the temperature threshold), the vent 116 may move from the closed position 122 to the open position 120 to release the vapor 118 and reduce the pressure inside the battery. In this regard, the release of the vapor 118 may indicate that the battery is not operating normally (e.g., the battery is overheating and going into thermal runaway).

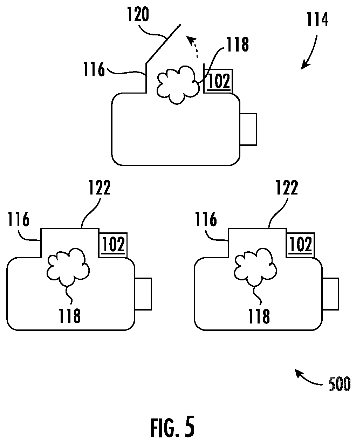

Although the sensor 102 and the one or more batteries 114 are depicted as separate components in FIG. 1, it would be understood by one skilled in the field to which this disclosure pertains that, in some embodiments, the sensor 102 and the one or more batteries 114 may be combined into a single component. For example, the sensor 102 may be integrated into one of the one or more batteries (e.g., attached to a surface of the one of the one or more batteries 114). Additionally, or alternatively, although only one sensor 102 is depicted in FIG. 1, it would be understood by one skilled in the field to which this disclosure pertains that, in some embodiments, the system 100 for detecting a vapor 118 may include more than one sensor 102. For example, as depicted in FIG. 5, the system 100 for detecting a vapor 118 may include a sensor 102 for each of the one or more batteries 114. As another example, the system 100 for detecting a vapor 118 may include a first sensor 102 for a portion of the one or more batteries 114 and a second sensor 102 for another portion of the one or more batteries 114.

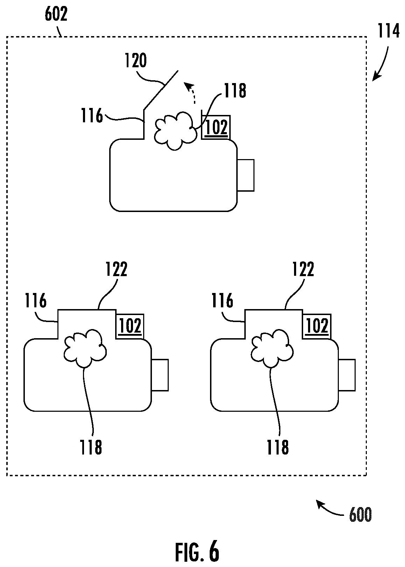

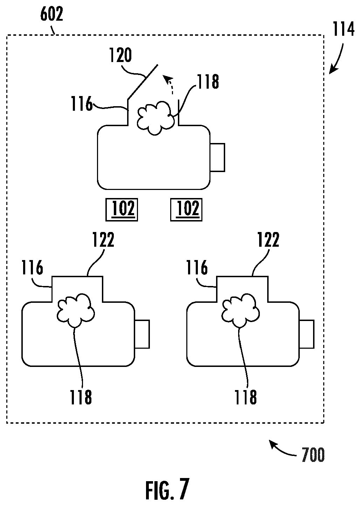

In some embodiments, such as depicted in FIGS. 6-8, the one or more batteries 114 may be organized into one or more battery packs 602. In some embodiments, for example, each of the one or more batteries 114 in a battery pack 602 may be associated with a sensor 102. For example, a sensor 102 may be disposed proximate to each of the one or more batteries 114 (e.g., a sensor 102 is attached to the surface of each of the one or more batteries 114). In some embodiments, for example, each of the one or more batteries 114 in a battery pack 602 may not be associated with a sensor 102. For example, a battery pack 602 having three batteries 114, may have two sensors 102 with each of the two sensors 102 associated with some or all of the batteries 114 in the battery pack 602 (e.g., each sensor 102 may be associated with batteries 114 proximate the sensor 102 in the battery pack 602). In some embodiments, the number of sensors 102 in the battery pack 602 may depend on the size of the battery pack 602 (e.g., the number of batteries 114 in the battery pack 602, the physical dimensions of the battery pack 602, etc.). In this regard, for example, the greater the size of the battery pack 602, the greater the number of sensors 102 in the battery pack 602.

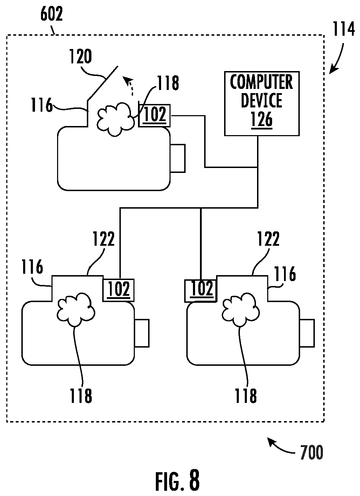

In some embodiments, such as depicted in FIG. 8, the one or more battery packs 602 may include a computing device, such as computing device 126. In this regard, for example, the computing device 126 may serve as battery pack management circuity for the battery pack 602. For example, the computing device 126 may be in communication with each of the sensors 102 in the battery pack 602. As described above, although the sensors 102 and the computing device 126 in battery pack 602 are depicted as separate components in FIG. 8, it would be understood by one skilled in the field to which this disclosure pertains that, in some embodiments, the sensors 102 and the computing device 126 may be combined into a single component. For example, the sensors 102 may integrated into the computing device 126 or the computing device 126 may be integrated into the sensor 102 (e.g., the sensors 102 may be integrated into the battery pack management circuity of the battery pack 602 or the battery pack management circuity of the battery pack 602 may be integrated into the sensors 102).

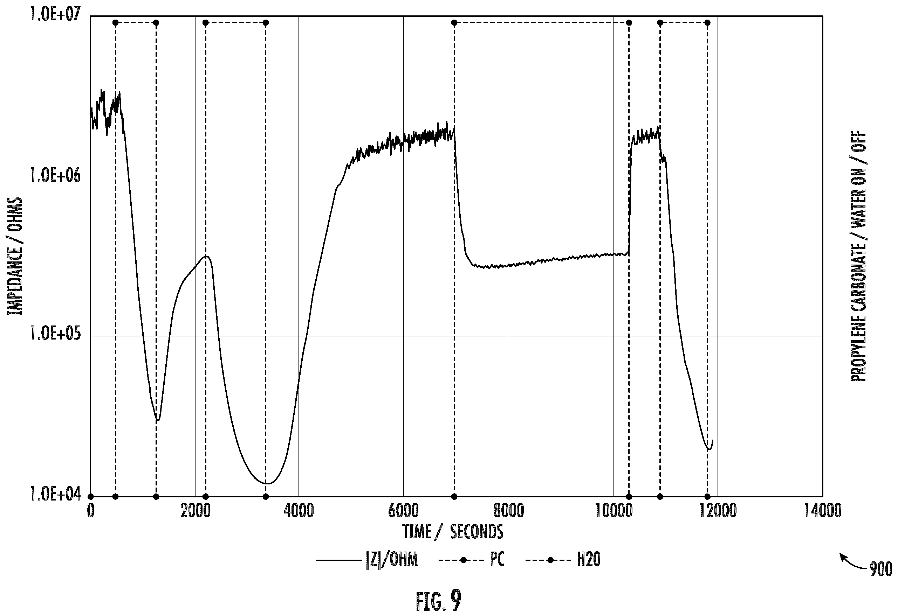

In some embodiments, such as depicted in FIG. 9, an impedance of one or more sensors 102 may be measured and/or graphed for a time instance and/or time interval (e.g., a time period). As shown, a graph 900 may provide a depiction of an impedance of the one or more sensors 102 for one or more time intervals when the one or more sensors 102 are exposed to one or more vapors 118. For example, the one or more sensors 102 may be exposed to an electrolyte vapor (e.g., PC) for a first duration (e.g., 500 seconds (s) to 1000 s), a second duration (e.g., 2250 s to 3250 s), and third duration (e.g., 11000 s to 11750 s). When the one or more sensors 102 are exposed to the electrolyte vapor, the impedance of the one or more sensors 102 may decrease by a first amount (e.g., at a first rate). In some examples, the one or more sensors 102 may be exposed to water vapor for a fourth duration (e.g., 7000 s to 10250 s). When the one or more sensors 102 are exposed to the water vapor, the impedance of the one or more sensors 102 may decrease by a second amount (e.g., at a second rate). In some examples, the second amount may be less than the first amount (e.g., the one or more sensors 102 may respond differently in response to being exposed to water vapor than in response to being exposed to an electrolyte vapor). In some examples, the second rate may be higher than the first rate.

In some examples, an impedance of a sensor 102 may be indicative of or otherwise based on a quantity of one or more vapors 118 that react with the sensor 102. For example, a first equation may specify a relationship between the impedance, a quantity of an electrolyte vapor (e.g., a first vapor) that has reacted with the sensor 102, and a quantity of water vapor (e.g., a second vapor) that has reacted with the sensor 102. In some examples, determining or otherwise measuring an impedance of the sensor 102 (e.g., by a computing device 126) may enable respective quantities of one or more vapors 118 to be determined (e.g., by the computing device 126). For example, the computing device 126 may solve a system of equations including the first equation and a second equation that relates the respective quantities of the one or more vapors 118 to one or more other characteristics of the sensor 102, such as a phase angle associated with the sensor 102.

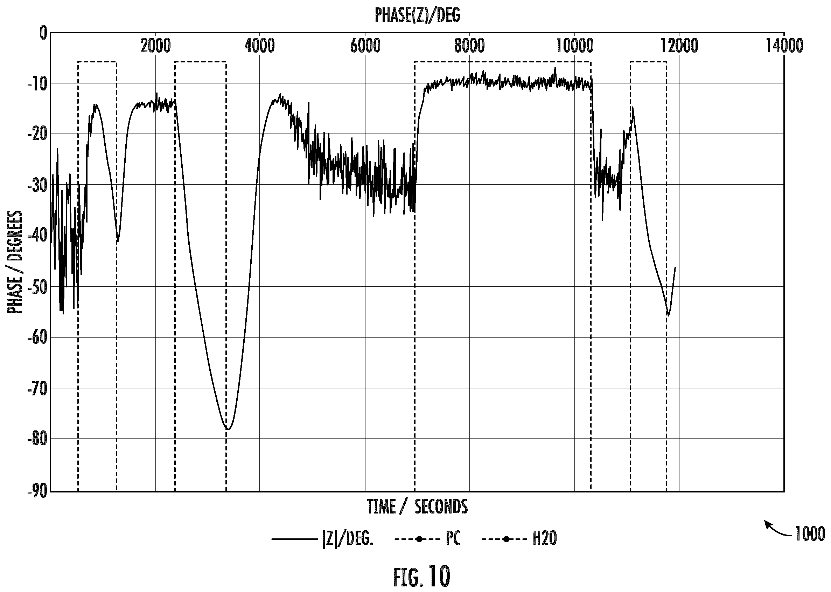

In some embodiments, such as depicted in FIG. 10, a capacitance of one or more sensors 102 may be measured and/or graphed for a time instance and/or time interval (e.g., a time period). As shown, a graph 900 may provide a depiction of a phase angle associated with one or more sensors 102 for one or more time intervals when the one or more sensors 102 are exposed to one or more vapors 118. As described herein, the phase angle may be indicative of or otherwise related to (e.g., proportional to) a capacitance of the one or more sensors 102. As shown, the one or more sensors 102 may be exposed to an electrolyte vapor (e.g., PC) for a first duration (e.g., 500 seconds (s) to 1000 s), a second duration (e.g., 2250 s to 3250 s), and third duration (e.g., 11000 s to 11750 s). When the one or more sensors 102 are exposed to the electrolyte vapor, the phase angle associated with the one or more sensors 102 may decrease (and capacitance may decrease) by a first amount (e.g., at a first rate). In some examples, the one or more sensors 102 may be exposed to water vapor for a fourth duration (e.g., 7000 s to 10250 s). When the one or more sensors 102 are exposed to the water vapor, the phase angle associated with the one or more sensors 102 may increase (and capacitance may increase) by a second amount (e.g., at a second rate). In some examples, the second amount may be less than the first amount (e.g., the one or more sensors 102 may respond differently in response to being exposed to water vapor than in response to being exposed to an electrolyte vapor). In some examples, the second rate may be higher than the first rate.

In some examples, a phase angle associated with a sensor 102 may be indicative of or otherwise based on a quantity of one or more vapors 118 that react with the sensor 102. For example, a second equation may specify a relationship between the phase angle, a quantity of an electrolyte vapor (e.g., a first vapor) that has reacted with the sensor 102, and a quantity of water vapor (e.g., a second vapor) that has reacted with the sensor 102. In some examples, determining or otherwise measuring the phase angle associated with the sensor 102 (e.g., by a computing device 126) may enable respective quantities of one or more vapors 118 to be determined (e.g., by the computing device 126). For example, the computing device 126 may solve a system of equations including the first equation and the second equation.

Although some examples described herein refer to multiple vapors 118 being exposed to and/or reacting with a sensor 102 during different time intervals, two or more vapors 118 may additionally, or alternatively be exposed to and/or react with a sensor 102 during a same time interval or during partially overlapping time intervals. For example, the sensor 102 may continuously (e.g., for a first duration) be exposed to and/or react with water vapor (e.g., at varying levels). During the first duration, the sensor 102 may be exposed to and/or react with an electrolyte vapor (e.g., at varying levels).

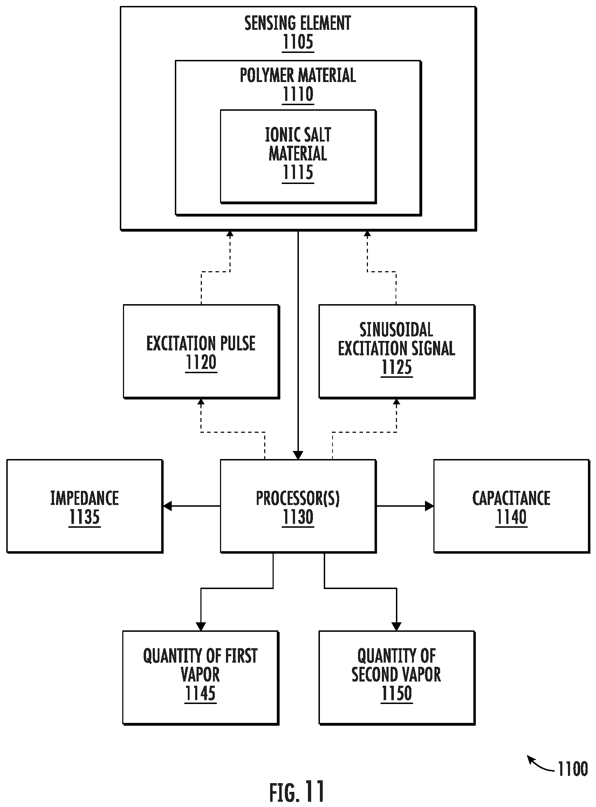

In some embodiments, such as depicted in FIG. 11, any one or more of the techniques described herein may be performed in accordance with a dataflow diagram 1100. The dataflow diagram 1100 shows example data structures and modules for determining quantities of multiple vapors using a single polymer sensor. In some examples, an impedance 1135 of a sensing element 1105 (e.g., a polymer support 106 and/or an ionic salt 108, a sensor 102) may be determined. For example, one or more processors 1130 (e.g., of a computing device 126) may determine the impedance 1135 of a sensing element 1105. In some examples, a capacitance 1140 of the sensing element 1105 may be determined. For example, the one or more processors 1130 may determine the capacitance 1140 of the sensing element 1105. In some embodiments, one or more processors 1130 may be one or more components or devices that are configurable to perform one or more operations, calculations, determinations, communications, or logical processes. In some examples, a processor 1130 may be a subcomponent of a computing device. In some other examples, however, one or more processors 1130 may be implemented as virtualized elements of a virtualized computing system or architecture. In some examples, the one or more processors 1130 may be included in a sensor device and/or sensor system. For example, a gas sensing device may include one or more processors 1130 and/or one or more sensing elements 105 in communication with the one or more processors 1130. The one or more processors 1130 may be configured to perform any one or more of the operations described herein.

In some embodiments, a sensing element 1105 may be a component configured to sense one or more phenomena. For example, a sensing element 1105 may exhibit a response to a phenomenon, which may be measured and/or quantified. In some examples, a sensing element 1105 may be a composite material or structure. For example, a sensing element 1105 may include a polymer (e.g., a polymer film) and an ionic salt that is embedded in or layered onto the polymer film. In some examples, one or more properties and/or characteristics of a sensing element 1105 may change in response to one or more phenomena and/or stimuli. For example, a conductivity of a sensing element 1105 may increase or decrease when one or more vapors react with the sensing element 1105. Additionally, or alternatively, a capacitance 1140 of the sensing element 1105 may increase or decrease when one or more vapors react with the sensing element 1105. As described herein, the terms "sensing element" and "sensor" may be used interchangeably without loss of meaning.

In some embodiments, an impedance 1135 may be a measure or value representative of the opposition to alternating current. For example, an electric circuit and/or component may exhibit or otherwise be associated with an impedance 1135. In some examples, impedance 1135 may arise from the combined effect of ohmic resistance and reactance. In one example of a circuit element coupled with two terminals (e.g., a sensing element 1105), an impedance value may be calculated as a ratio of a complex representation of a sinusoidal voltage between the terminals to a complex representation of the current flowing through the circuit element.

As described herein, an impedance value (e.g., of a sensing element 1105) may be determined by one or more processors 1130. In such examples, the one or more processors 1130 may cause an excitation signal (e.g., a sinusoidal signal, a pulse) to be applied to a sensing element 1105 (e.g., by one or more signal generation components). For example, the one or more processors 1130 may transmit one or more control signals to one or more signal generation components, which may generate and/or transmit one or more excitation signals to the sensing element 1105 in response to receiving the one or more control signals. In some examples, the one or more processors 1130 may then measure the impedance 1135 or otherwise cause the impedance 1135 to be measured (e.g., by comparing the excitation signal to a signal output by the sensing element 1105).

In some examples, an impedance value of a sensing element 1105 may be indicative of or representative of a quantity of a vapor that has reacted with or otherwise been exposed to the sensing element 1105. For example, an electrolyte vapor may react with one or more materials of a sensing element 1105 and cause an impedance 1135 of the sensing element 1105 to decrease. Additionally, or alternatively, a conductivity of the sensing element 1105 may rise in response to the electrolyte vapor reacting with the one or more materials of the sensing element 1105. In some examples, water vapor may additionally, or alternatively cause an impedance 1135 of the sensing element 1105 to decrease. However, the sensing element 1105 may be less sensitive to water vapor than electrolyte vapor. Accordingly, the impedance 1135 of the sensing element 1105 may decrease more in the presence of electrolyte vapor than in the presence of water vapor.

In some embodiments, a capacitance 1140 may be a value representative of an object, material, and/or electrical component's ability to store electric charge. For example, a component of an electrical circuit, such as a sensing element 1105, may exhibit or otherwise be associated with a capacitance 1140. As described herein, a capacitance value (e.g., of a sensing element 1105) may be determined by one or more processors 1130. In such examples, the one or more processors 1130 may cause an excitation signal (e.g., a sinusoidal signal, a pulse) to be applied to a sensing element 1105 (e.g., by one or more signal generation components). For example, the one or more processors 1130 may transmit one or more control signals to one or more signal generation components, which may generate and/or transmit one or more excitation signals to the sensing element 1105 in response to receiving the one or more control signals. In some examples, the one or more processors 1130 may then measure the capacitance 1140 or otherwise cause the capacitance 1140 to be measured (e.g., by comparing the excitation signal to a signal output by the sensing element 1105). In some examples, a phase angle associated with the excitation signal may be indicative of a capacitance 1140 of the sensing element 1105. For example, a phase angle of an impedance 1135 measurement may be indicative of a capacitance 1140 of the sensing element 1105 (e.g., a phase angle between an input excitation signal and an output signal output by a sensing element 1105).

In some examples, a capacitance value of a sensing element 1105 may be indicative of or representative of a quantity of a vapor that has reacted with or otherwise been exposed to the sensing element 1105. For example, water vapor may react with one or more materials of a sensing element 1105 and cause a capacitance 1140 of the sensing element 1105 to increase or decrease. In some examples, an electrolyte vapor may additionally, or alternatively cause a capacitance 1140 of the sensing element 1105 to increase or decrease. However, the sensing element 1105 may be less sensitive to the electrolyte vapor than water vapor. Accordingly, the capacitance 1140 of the sensing element 1105 may increase or decrease more in the presence of water vapor than in the presence of the electrolyte vapor.

In some examples, a quantity of a first vapor 1145 that has reacted with the sensing element 1105 and a quantity of a second vapor 1150 that has reacted with the sensing element 1105 may be determined. For example, the one or more processors 1130 may determine the quantity of the first vapor and the quantity of the second vapor. In some examples, the determination and/or the quantities may be based on the impedance 1135 of the sensing element 1105 and the capacitance 1140 of the sensing element 1105.

In some embodiments, a vapor may be a substance or phase of matter, which may be airborne or otherwise suspended. A vapor may be a substance that is in a gaseous state. In some examples, a vapor may react with one or more other substances or materials. For example, a sensing element 1105 (e.g., one or more materials of a sensing element 1105) may absorb one or more vapors. In some examples, a vapor may be emitted by a device or substance. For example, a battery may emit an electrolyte vapor (e.g., a first vapor) in a specific scenario or context. In some examples, a battery may emit an electrolyte vapor in one or more failure modes (e.g., if thermal runaway is occurring at the battery). In such examples, the presence of an electrolyte vapor and/or a specific quantity of an electrolyte vapor (e.g., a threshold quantity) may be indicative of a battery experiencing thermal runaway.

In some embodiments, a quantity may be an amount, volume, or concentration of a substance, such as a vapor. A quantity may be expressed as a value with reference to one or more units of measure. For example, a quantity of a vapor may be expressed as a quantity of milliliters (mLs), moles (mols), and/or the like. In some examples, one or more properties or characteristics of a sensing element 1105 may be indicative of a quantity of one or more vapors that have reacted with the sensing element 1105. For example, an impedance 1135 of a sensing element 1105 may be indicative of a quantity of water vapor and/or a quantity of electrolyte vapor that have reacted with the sensing element 1105.

In some embodiments, a reaction may be a process or interaction between any combination of two or more substances, materials, or compounds. For example, a vapor that comes into contact with a sensing element 1105 may cause one or more reactions to occur (e.g., an ionic salt of the sensing element 1105 may solvate). Accordingly, the vapor may be said to react with the sensing element 1105. In some examples, a reaction may include one or more absorption processes. For example, a reaction may include a sensing element 1105 absorbing one or more vapors.

In some examples, the first vapor includes an electrolyte vapor and the second vapor includes water vapor. As described herein, an electrolyte vapor may include, refer to, or otherwise be an example of one or more types of electrolyte vapors. For example, some non-limiting examples of electrolyte vapors may include propylene carbonate (PC), ethylene carbonate (EC), diethyl carbonate (DEC), dimethyl carbonate (DMC), ethyl methyl carbonate (EMC), dimethoxyethane (DME), gamma-butyrolactone (GBL), and/or the like.

In some examples, a sensing element 1105 may be exposed to and/or react with two or more vapors (e.g., simultaneously). For example, a sensing element 1105 may react with an electrolyte vapor (e.g., a first vapor) and water vapor (e.g., humidity, a second vapor). In such examples, the techniques described herein may enable one or more processors 1130 to determine quantities of both vapors that have reacted with the sensing element 1105. In some examples, the sensing element 1105 includes a polymer material 1110 (e.g., a polymer support 106) and an ionic salt material 1115 (e.g., an ionic salt 108).

In some examples, a sinusoidal excitation signal 1125 having a frequency that is based at least in part on one or more properties of the sensing element 1105 may be transmitted to and/or towards the sensing element 1105. In some examples, the one or more processors 1130 may cause transmission of the sinusoidal excitation signal 1125. In some examples, determining the impedance 1135 of the sensing element 1105 is based on the sinusoidal excitation signal 1125.

In some examples, an excitation signal and/or an excitation pulse 1120 (e.g., an electrical signal and/or pulse) may be transmitted towards and/or through a sensing element 1105. For example, a first and second electrode (e.g., conductor) may be coupled with the sensing element 1105. In such examples, an excitation signal and/or an excitation pulse 1120 may be routed through the sensing element 1105 via the first electrode and the second electrode (e.g., the electrical signal may enter the sensing element 1105 through the first electrode and exit the sensing element 1105 through the second electrode). In some examples, one or more characteristics of an excitation signal may be selected (e.g., by a user, by a computing device) based on one or more properties of the sensing element 1105. For example, a frequency of an excitation signal (e.g., an alternating current (AC) electrical signal) may be selected based on a quantity of an ionic salt material 1115 in the sensing element 1105. In such examples, the one or more characteristics of the excitation signal may be selected to elicit a specific or known response by the sensing element 1105.

In some embodiments, an excitation signal may be an electrical signal that is used to determine one or more characteristics of a sensing element 1105. For example, an impedance 1135 and/or a capacitance 1140 of a sensing element 1105 may be determined based on an excitation signal. An excitation signal may be a digital or analog signal. In some examples, an excitation signal may be a waveform, such as a sinusoidal excitation signal 1125, a sawtooth excitation signal, a square wave excitation signal, and/or the like. In some examples, an excitation signal may include one or more pulses. In some examples, an excitation signal may be generated by one or more processors 1130 or a signal generation component that is controlled by the one or more processors 1130. In some examples, an excitation signal may have a frequency, which may be selected by a user or the one or more processors 1130 to achieve a specific goal. For example, a frequency of an excitation signal may selected based on test results that indicate an optimal frequency for the excitation signal (e.g., a frequency that results in a more pronounced response by a sensing element 1105 when compared to one or more other frequencies that are tested).

In some examples, an excitation signal may be transmitted to and/or across a sensing element 1105. In such examples, a response of the sensing element 1105 may be measured, which may be indicative of a quantity of one or more vapors that have reacted with the sensing element 1105. For example, the response of the sensing element 1105 may indicate an impedance 1135 of the sensing element 1105 and/or a phase angle of the sensing element 1105 (e.g., a capacitance 1140 of the sensing element 1105).

Although some examples described herein refer to measuring impedance 1135 and/or capacitance 1140 of a sensing element 1105 using an excitation signal with a specific frequency, one or more variable frequency approaches may be utilized. For example, a spectroscopy approach may be utilized in which a plurality of excitation signals having a plurality of frequencies are transmitted to a sensing element 1105. In some examples, a frequency scanning operation may be utilized to modulate an excitation signal (e.g., scan through a plurality of frequencies), which may enable one or more quantities of one or more vapors to be determined based on a frequency response of the sensing element 1105 to the modulated excitation signal.

In some examples, determining the quantity of the first vapor and/or the quantity of the second vapor may include solving, by the one or more processors 1130, a first equation and/or a second equation. The first equation may specify a relationship between the impedance 1135 of the sensing element 1105, the quantity of the first vapor, and the quantity of the second vapor. The second equation may specify a relationship between the capacitance 1140 of the sensing element 1105, the quantity of the first vapor, and the quantity of the second vapor.

In some embodiments, an equation may be a statement that the values of two mathematical expressions are equal. As described herein, a first equation may relate a first characteristic (e.g., an impedance 1135) of a sensing element 1105 to a quantity of a first vapor 1145 (e.g., an electrolyte vapor) that has reacted with the sensing element 1105 and a quantity of a second vapor 1150 (e.g., water vapor) that has reacted with the sensing element 1105. A second equation may relate a second characteristic (e.g., a phase angle of the impedance 1135, a capacitance 1140) of the sensing element 1105 to the quantity of the first vapor that has reacted with the sensing element 1105 and the quantity of the second vapor that has reacted with the sensing element 1105. In some examples, the first characteristic and the second characteristic may be determined or otherwise measured (e.g., by one or more processors 1130). The one or more processors 1130 may then perform one or more operations and/or calculations to determine the quantity of the first vapor and the quantity of the second vapor based on the first characteristic and the second characteristic. For example, the one or more processors 1130 may solve (e.g., algebraically) the system of equations including the first equation and the second equation.

In some examples, the one or more processors 1130 may determine that the quantity of the first vapor satisfies a threshold quantity. In some examples, the one or more processors 1130 may cause a message to be displayed via a user interface. The message may alert a user that the quantity of the first vapor satisfies the threshold quantity. In some embodiments, a threshold quantity may be a value, level, or limit that serves as a reference or trigger for performing one or more actions and/or operations. A threshold quantity may be an upper limit or a lower limit. In some examples, a threshold quantity may be satisfied when a value and/or a quantity is greater than, less than, or equal to the threshold quantity. As described herein, one or more processors 1130 may determine if a quantity of a vapor satisfies a threshold quantity (e.g., a threshold vapor quantity). In such examples, the threshold quantity may be a quantity of a vapor (e.g., an electrolyte vapor) that is indicative of a failure mode of a battery or a quantity of vapor that otherwise presents one or more hazards. In some examples, the threshold quantity may be satisfied if a measured quantity of the vapor is greater than or equal to the threshold quantity.

In some embodiments, a user interface may be hardware and/or software that is configured to interface with one or more individuals (e.g., one or more users). For example, a user interface may be a device that receives one or more inputs from a user and/or provides one or more outputs to the user, such as a monitor, a display, a speaker, a microphone, a printer, a keyboard, a mouse, a joystick, and/or the like. In some examples, a user interface may be a software application, such as a graphical user interface that is displayed and/or executed on a computing device. In some examples, a user interface may provide an audio and/or visual representation of information. For example, a user interface of a computing device, such as a smartphone, may display one or more images, which may be viewed and/or interacted with by one or more individuals (e.g., via a touchscreen, via one or more buttons). In some examples, an image displayed via a user interface may include a text string, which may be representative of a text string located on an object in a real-world environment (e.g., an image displayed on a user interface may include a representation of a text string located on a shipping container, a package, a product, a document (e.g., an identification document), and/or the like).

In some examples, the one or more processors 1130 may cause a safety protocol to be performed based on the quantity of the first vapor. In some examples, the safety protocol includes one or more of venting a battery and/or disconnecting the battery. In some embodiments, a safety protocol may be a procedure or operation that is performed to achieve one or more safety outcomes. For example, one or more processors 1130 may initiate or otherwise cause one or more safety protocols to be performed. A safety protocol may include deactivating, deenergizing, and/or disconnecting one or more electrical components. For example, a safety protocol may include disconnecting a battery from one or more devices (e.g., a battery in an electric vehicle may be disconnected from an electric motor or any other electrical component that draws current from the battery). In some examples, a safety protocol may include performing one or more operations to vent one or more vapors from a battery and/or a compartment that stores a battery. For example, one or more processors 1130 may send a control signal to an electronically actuated venting component that vents a battery in response to a determination that an electrolyte vapor quantity satisfies a threshold quantity.

In some examples, the quantity of the first vapor may be determined based on the sinusoidal excitation signal 1125 applied to the sensing element 1105. In some examples, the one or more processors 1130 may cause transmission of an excitation pulse 1120 to the sensing element 1105. The one or more processors 1130 may then determine a second quantity of the first vapor based on the excitation pulse 1120. The one or more processors 1130 may then determine an error value associated with the second quantity of the first vapor based at least in part on a comparison of the second quantity of the first vapor and the quantity of the first vapor.

In some embodiments, a pulse may be a type of electrical signal, such as an excitation signal (e.g., an excitation pulse 1120). For example, a pulse may be a first type of excitation signal and a sinusoidal excitation signal 1125 may be a second type of excitation signal. In some examples, a pulse may be a type of direct current (DC) signal and a sinusoidal signal may be a type of AC signal. In some examples, a waveform may include one or more pulses, which may each have pulse widths.

In some examples, a pulse may be utilized to determine one or more characteristics of a sensing element 1105. For example, one or more processors 1130 may cause the transmission of a pulse to a sensing element 1105. An impedance 1135, capacitance 1140, and/or time decay may then be determined based on the pulse. As described herein, a sinusoidal excitation signal 1125 may additionally, or alternatively be utilized to determine the one or more characteristics of the sensing element 1105. However, generating a sinusoidal excitation signal 1125 (or any other type of AC excitation signal) may be more costly than generating a pulse excitation signal. For example, one or more hardware components for generating a sinusoidal excitation signal 1125 may be more costly than one or more hardware components for generating a pulse excitation signal. Additionally, or alternatively, various signal processing operations may be more resource intensive for sinusoidal excitation signals 1125 when compared to pulse excitation signals.

Accordingly, in one example embodiment of the present disclosure, one or more pulse excitation signals (e.g., one or more excitation pulses 1120) may be utilized to determine one or more vapor quantities during a first time period and one or more sinusoidal excitation signals 1125 may be utilized to determine one or more vapor quantities during a second time period that is shorter than the first time period. Such techniques may enable the selective utilization of more computationally intensive sensing operations (e.g., using sinusoidal excitation signals 1125) to achieve more accurate vapor sensing results that would not otherwise be achievable with less computationally intensive sensing operations.

In some examples, a vapor sensing operation that utilizes an AC excitation signal may be performed if a quantity of a specific vapor is determined to have satisfied a threshold quantity based on a vapor sensing operation that utilizes a DC excitation signal (e.g., an excitation pulse 1120). For example, a first type of vapor sensing operation (e.g., that utilizes a DC excitation pulse 1120, a lower cost sensing operation) may be performed periodically and/or continuously. If a quantity of a vapor (e.g., an electrolyte vapor) that is detected using the first type of vapor sensing operation satisfies a threshold quantity, a second type of vapor sensing operation may be triggered (e.g., as a verification step, to determine an error value and/or validity of the previously detected vapor quantity). The second type of vapor sensing operation may be more costly than the first type of vapor sensing operation. However, the second type of vapor sensing operation (e.g., a vapor sensing operation using a sinusoidal excitation signal 1125 and/or a spectroscopy-based vapor sensing operation) may be more accurate than the first type of vapor sensing operation. For example, the second type of vapor sensing operation may be capable of correcting error in the first type of vapor sensing operation associated with the presence of humidity, which may result in improved accuracy of electrolyte vapor measurements.

In some embodiments, an error value may be a value indicative of a degree of inaccuracy associated with a measurement. In some examples, an error value may indicate a difference between a ground-truth value and a measured value. In some examples, an error value may indicate a difference between a value that is measured or otherwise determined using a first vapor sensing operation and a value that is measured or otherwise determined using a second vapor sensing operation. For example, an error value may be indicative of a difference between a vapor quantity that is determined using an AC excitation signal and a vapor quantity that is determined using a DC excitation signal.

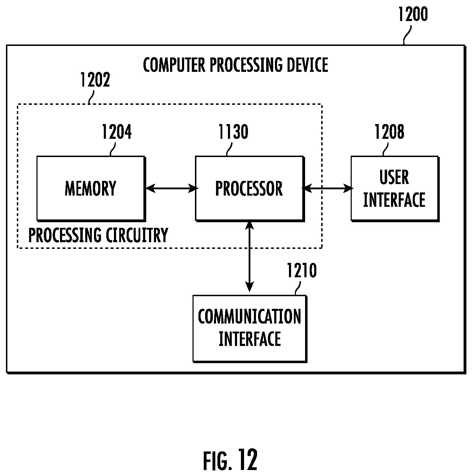

With reference to FIG. 12, a block diagram of an example computer processing device 1200 is illustrated in accordance with some example embodiments. In some embodiments, the computing device 126 (e.g., the battery pack management circuitry, the battery management circuitry, etc.) and/or other devices may be embodied as one or more computer processing devices, such as the computer processing device 1200 in FIG. 12. However, it should be noted that the components, devices, or elements illustrated in and described with respect to FIG. 12 below may not be mandatory and thus one or more may be omitted in certain embodiments. Additionally, some embodiments may include further or different components, devices or elements beyond those illustrated in and described with respect to FIG. 12.

The computer processing device 1200 may include or otherwise be in communication with processing circuitry 1202 that is configurable to perform actions in accordance with one or more embodiments disclosed herein. In this regard, the processing circuitry 1202 may be configured to perform and/or control performance of one or more functionalities of the computer processing device 1200 in accordance with various embodiments, and thus may provide means for performing functionalities of the computer processing device 1200 in accordance with various embodiments. The processing circuitry 1202 may be configured to perform data processing, application execution and/or other processing and management services according to one or more embodiments. In some embodiments, the computer processing device 1200 or a portion(s) or component(s) thereof, such as the processing circuitry 1202, may be embodied as or comprise a chip or chip set. In other words, the computer processing device 1200 or the processing circuitry 1202 may comprise one or more physical packages (e.g., chips) including materials, components and/or wires on a structural assembly (e.g., a baseboard). The structural assembly may provide physical strength, conservation of size, and/or limitation of electrical interaction for component circuitry included thereon. The computer processing device 1200 or the processing circuitry 1202 may therefore, in some cases, be configured to implement an embodiment of the disclosure on a single chip or as a single "system on a chip." As such, in some cases, a chip or chipset may constitute means for performing one or more operations for providing the functionalities described herein.

In some embodiments, the processing circuitry 1202 may include one or more processors 1130 and, in some embodiments, such as that illustrated in FIG. 12, may further include memory 1204. The processing circuitry 1202 may be in communication with or otherwise control a user interface 1208 and/or a communication interface 1210. As such, the processing circuitry 1202 may be embodied as a circuit chip (e.g., an integrated circuit chip) configured (e.g., with hardware, software or a combination of hardware and software) to perform operations described herein.

A processor 1130 may be embodied in a number of different ways. For example, the processor 1130 may be embodied as various processing means such as one or more of a microprocessor or other processing element, a coprocessor, a controller or various other computing or processing devices including integrated circuits such as, for example, an ASIC (application specific integrated circuit), an FPGA (field programmable gate array), or the like. Although illustrated as a single processor, it will be appreciated that the processor 1130 may comprise a plurality of processors. The plurality of processors may be in operative communication with each other and may be collectively configured to perform one or more functionalities of the computer processing device 1200 as described herein. In some embodiments, the processor 1130 may be configured to execute instructions stored in the memory 1204 or otherwise accessible to the processor 1130. As such, whether configured by hardware or by a combination of hardware and software, the processor 1130 may represent an entity (e.g., physically embodied in circuitry - in the form of processing circuitry 1202) capable of performing operations according to embodiments of the present disclosure while configured accordingly. Thus, for example, when the processor 1130 is embodied as an ASIC, FPGA or the like, the processor 1130 may be specifically configured hardware for conducting the operations described herein. Alternatively, as another example, when the processor 1130 is embodied as an executor of software instructions, the instructions may specifically configure the processor 1130 to perform one or more operations described herein.

In some embodiments, the memory 1204 may include one or more non-transitory memory devices such as, for example, volatile and/or non-volatile memory that may be either fixed or removable. In this regard, the memory 1204 may comprise a non-transitory computer-readable storage medium. It will be appreciated that while the memory 1204 is illustrated as a single memory, the memory 1204 may comprise a plurality of memories. The memory 1204 may be configured to store information, data, applications, instructions and/or the like for enabling the computer processing device 1200 to carry out various functions in accordance with one or more embodiments. For example, the memory 1204 may be configured to buffer input data for processing by the processor 1130. Additionally, or alternatively, the memory 1204 may be configured to store instructions for execution by the processor 1130. As yet another alternative, the memory 1204 may include one or more databases that may store a variety of files, contents or data sets. Among the contents of the memory 1204, applications may be stored for execution by the processor 1130 in order to carry out the functionality associated with each respective application. In some cases, the memory 1204 may be in communication with one or more of the processor 1130, user interface 1208, and/or communication interface 1210 via a bus(es) for passing information among components of the computer processing device 1200.

The user interface 1208 may be in communication with the processing circuitry 1202 to receive an indication of a user input at the user interface 1208 and/or to provide an audible, visual, mechanical or other output to the user. As such, the user interface 1208 may include, for example, a keyboard, a mouse, a joystick, a display, a touch screen display, a microphone, a speaker, and/or other input/output mechanisms. As such, the user interface 1208 may, in some embodiments, provide means for a user to access and interact with the computing device 126 and/or the sensor 102.

The communication interface 1210 may include one or more interface mechanisms for enabling communication with other devices and/or networks. In some cases, the communication interface 1210 may be any means such as a device or circuitry embodied in either hardware, or a combination of hardware and software that is configured to receive and/or transmit data from/to a network and/or any other device or module in communication with the processing circuitry 1202. By way of example, the communication interface 1210 may be configured to enable the computing device 126 to communicate with the sensor 102 and/or other computing devices. Accordingly, the communication interface 1210 may, for example, include an antenna (or multiple antennas) and supporting hardware and/or software for enabling communications with a wireless communication network (e.g., a wireless local area network, cellular network, global positing system network, and/or the like) and/or a communication modem or other hardware/software for supporting communication via cable, digital subscriber line (DSL), universal serial bus (USB), Ethernet or other methods.

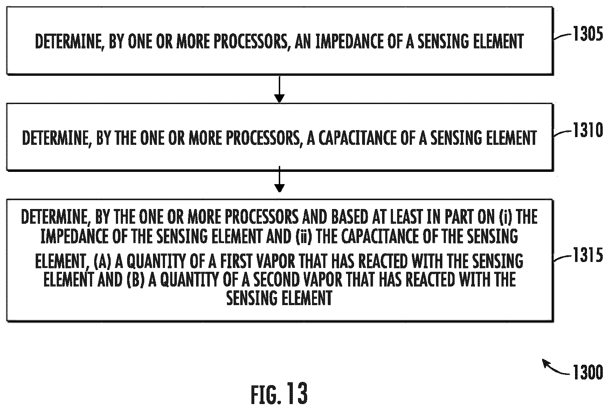

With reference to FIG. 13, a process 1300 for determining quantities of multiple vapors using a single sensor is illustrated in accordance with some example embodiments. Specifically, FIG. 13 depicts operations of an example process 1300. In some embodiments, the process 1300 is embodied by computer program code stored on a non-transitory computer-readable storage medium of a computer program product configured for execution to perform the process as depicted and described. Additionally, or alternatively, in some embodiments, the process 1300 is performed by at least one specially configured computing device, such as at least one computer processing device 1200 alone or in communication with at least one other component, device, system, and/or the like. In this regard, in some such embodiments, the computer processing device 1200 is specially configured by computer-coded instructions (e.g., computer program instructions) stored thereon, for example in the memory 1204 and/or another component depicted and/or described herein and/or otherwise accessible to the computer processing device 1200, for performing the operations as depicted and described. In some embodiments, the computer processing device 1200 is in communication with at least one external apparatus, system, device, and/or the like, to perform at least one of the operations as depicted and described. For example, the computer processing device 1200, in some embodiments, is in communication with an external computing device, a client device, and/or the like. For purposes of simplifying the description, the process 1300 is described as performed by and from the perspective of the computer processing device 1200.

The process 1300 begins at operation 1305. At operation 1305, the computer processing device 1200 includes means such as the one or more processors 1130, the memory 1204, the communication interface 1210, the user interface 1208, or a combination thereof, to determine, by the one or more processors 1130, an impedance of a sensing element.

At operation 1310, the computer processing device 1200 includes means such as the one or more processors 1130, the memory 1204, the communication interface 1210, the user interface 1208, or a combination thereof, to determine, by the one or more processors 1130, a capacitance of the sensing element.

At operation 1315, the computer processing device 1200 includes means such as the one or more processors 1130, the memory 1204, the communication interface 1210, the user interface 1208, or a combination thereof, to determine, by the one or more processors and based at least in part on the impedance of the sensing element and the capacitance of the sensing element, a quantity of a first vapor that has reacted with the sensing element and a quantity of a second vapor that has reacted with the sensing element.

Many modifications and other embodiments of the inventions set forth herein will come to mind to one skilled in the art to which these inventions pertain having the benefit of teachings presented in the foregoing descriptions and the associated drawings. Although the figures only show certain components of the apparatus and systems described herein, it is understood that various other components may be used in conjunction with the system. Therefore, it is to be understood that the inventions are not to be limited to the specific embodiments disclosed and that modifications and other embodiments are intended to be included within the scope of the appended claims. Moreover, the steps in the method described above may not necessarily occur in the order depicted in the accompanying diagrams, and in some cases one or more of the steps depicted may occur substantially simultaneously, or additional steps may be involved. Although specific terms are employed herein, they are used in a generic and descriptive sense only and not for purposes of limitation.

While various embodiments in accordance with the principles disclosed herein have been shown and described above, modifications thereof may be made by one skilled in the art without departing from the spirit and the teachings of the disclosure. The embodiments described herein are representative only and are not intended to be limiting. Many variations, combinations, and modifications are possible and are within the scope of the disclosure. Alternative embodiments that result from combining, integrating, and/or omitting features of the embodiment(s) are also within the scope of the disclosure. Accordingly, the scope of protection is not limited by the description set out above.

Additionally, the section headings used herein are provided for consistency with the suggestions under 37 C.F.R. 1.77 or to otherwise provide organizational cues. These headings shall not limit or characterize the invention(s) set out in any claims that may issue from this disclosure.

Use of broader terms such as "comprises," "includes," and "having" should be understood to provide support for narrower terms such as "consisting of," "consisting essentially of," and "comprised substantially of" Use of the terms "optionally," "may," "might," "possibly," and the like with respect to any element of an embodiment means that the element is not required, or alternatively, the element is required, both alternatives being within the scope of the embodiment(s). Also, references to examples are merely provided for illustrative purposes, and are not intended to be exclusive.

### CONCLUSION

Many modifications and other embodiments of the disclosure set forth herein will come to mind to one skilled in the art to which this disclosure pertains having the benefit of the teachings presented in the foregoing description and the associated drawings. Therefore, it is to be understood that the embodiments are not to be limited to the specific embodiments disclosed and that modifications and other embodiments are intended to be included within the scope of the appended claims. Moreover, although the foregoing descriptions and the associated drawings describe example embodiments in the context of certain example combinations of elements and/or functions, it should be appreciated that different combinations of elements and/or functions may be provided by alternative embodiments without departing from the scope of the appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

In some embodiments, some of the operations above may be modified or further amplified. Furthermore, in some embodiments, additional optional operations may be included. Modifications, amplifications, or additions to the operations above may be performed in any order and in any combination.

Although an example processing system has been described above, implementations of the subject matter and the functional operations described herein can be implemented in other types of digital electronic circuitry, or in computer software, firmware, or hardware, including the structures disclosed in this specification and their structural equivalents, or in various combinations.

Embodiments of the subject matter and the operations described herein can be implemented in digital electronic circuitry, or in computer software, firmware, or hardware, including the structures disclosed in this specification and their structural equivalents, or in various combinations. Embodiments of the subject matter described herein can be implemented as at least one computer program, i.e., at least one module of computer program instructions, encoded on computer storage medium for execution by, or to control the operation of, information/data processing apparatus. Alternatively, or in addition, the program instructions can be encoded on an artificially generated, propagated signal, e.g., a machine-generated electrical, optical, or electromagnetic signal, which is generated to encode information/data for transmission to suitable receiver apparatus for execution by an information/data processing apparatus. A computer storage medium can be, or be included in, a computer-readable storage device, a computer-readable storage substrate, a random or serial access memory array or device, or a combination. Moreover, while a computer storage medium is not a propagated signal, a computer storage medium can be a source or destination of computer program instructions encoded in an artificially generated, propagated signal. The computer storage medium can also be, or be included in, at least one separate physical component or media (e.g., multiple CDs, disks, or other storage devices).

The operations described herein can be implemented as operations performed by an information/data processing apparatus on information/data stored on at least one computer-readable storage device or received from other sources.

The term "data processing apparatus" encompasses all kinds of apparatus, devices, and machines for processing data, including by way of example a programmable processor, a computer, a system on a chip, or multiple ones, or combinations, of the foregoing. The apparatus can include special purpose logic circuitry, e.g., an FPGA (field programmable gate array) or an ASIC (application-specific integrated circuit). The apparatus can also include, in addition to hardware, code that creates an execution environment for the computer program in question, e.g., code that constitutes processor firmware, a protocol stack, a repository management system, an operating system, a cross-platform runtime environment, a virtual machine, or any combination thereof. The apparatus and execution environment can realize various different computing model infrastructures, such as web services, distributed computing and grid computing infrastructures.

A computer program (also known as a program, software, software application, script, or code) can be written in any form of programming language, including compiled or interpreted languages, declarative or procedural languages, and it can be deployed in any form, including as a stand-alone program or as a module, component, subroutine, object, or other unit suitable for use in a computing environment. A computer program may, but need not, correspond to a file in a file system. A program can be stored in a portion of a file that holds other programs or information/data (e.g., at least one script stored in a markup language document), in a single file dedicated to the program in question, or in multiple coordinated files (e.g., files that store at least one module, subprogram, or portion of code). A computer program can be deployed to be executed on one computer or on multiple computers that are located at one site or distributed across multiple sites and interconnected by a communication network.

The processes and logic flows described herein can be performed by at least one programmable processor executing at least one computer program to perform actions by operating on input information/data and generating output. Processors suitable for the execution of a computer program include, by way of example, both general and special purpose microprocessors, and any processor of any kind of digital computer. Generally, a processor will receive instructions and information/data from a read-only memory or a random access memory or both. The essential elements of a computer are a processor for performing actions in accordance with instructions and at least one memory device for storing instructions and data. Generally, a computer will also include, or be operatively coupled to receive information/data from or transfer information/data to, or both, at least one mass storage device for storing data, e.g., magnetic, magneto-optical disks, or optical disks.

However, a computer need not have such devices. Devices suitable for storing computer program instructions and information/data include all forms of non-volatile memory, media and memory devices, including by way of example semiconductor memory devices, e.g., EPROM, EEPROM, and flash memory devices; magnetic disks, e.g., internal hard disks or removable disks; magneto-optical disks; and CD-ROM and DVD-ROM disks. The processor and the memory can be supplemented by, or incorporated in, special purpose logic circuitry.

To provide for interaction with a user, embodiments of the subject matter described herein can be implemented on a computer having a display device, e.g., a CRT (cathode ray tube) or LCD (liquid crystal display) monitor, for displaying information/data to the user and a keyboard and a pointing device, e.g., a mouse or a trackball, by which the user can provide input to the computer. Other kinds of devices can be used to provide for interaction with a user as well; for example, feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; for example, by sending web pages to a web browser on a user's client device in response to requests received from the web browser.

Embodiments of the subject matter described herein can be implemented in a computing system that includes a back-end component, e.g., as an information/data server, or that includes a middleware component, e.g., an application server, or that includes a front-end component, e.g., a client computer having a graphical user interface or a web browser through which a user can interact with an implementation of the subject matter described herein, or any combination of at least one such back-end, middleware, or front-end components. The components of the system can be interconnected by any form or medium of digital information/data communication, e.g., a communication network. Examples of communication networks include a local area network ("LAN") and a wide area network ("WAN"), an inter- network (e.g., the Internet), and peer-to-peer networks (e.g., ad hoc peer-to-peer networks).

The computing system can include clients and servers. A client and server are generally remote from each other and typically interact through a communication network. The relationship of client and server arises by virtue of computer programs running on the respective computers and having a client-server relationship to each other. In some embodiments, a server transmits information/data (e.g., an HTML page) to a client device (e.g., for purposes of displaying information/data to and receiving user input from a user interacting with the client device). Information/data generated at the client device (e.g., a result of the user interaction) can be received from the client device at the server.

While this specification contains many specific implementation details, these should not be construed as limitations on the scope of any disclosures or of what may be claimed, but rather as descriptions of features specific to particular embodiments of particular disclosures. Certain features that are described herein in the context of separate embodiments can also be implemented in combination in a single embodiment. Conversely, various features that are described in the context of a single embodiment can also be implemented in multiple embodiments separately or in any suitable sub combination. Moreover, although features may be described above as acting in certain combinations and even initially claimed as such, at least one feature from a claimed combination can in some cases be excised from the combination, and the claimed combination may be directed to a sub combination or variation of a sub combination.

Similarly, while operations are depicted in the drawings in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system components in the embodiments described above should not be understood as requiring such separation in all embodiments, and it should be understood that the described program components and systems can generally be integrated together in a single software product or packaged into multiple software products.

Thus, particular embodiments of the subject matter have been described. Other embodiments are within the scope of the following claims. In some cases, the actions recited in the claims can be performed in a different order and still achieve desirable results. In addition, the processes depicted in the accompanying figures do not necessarily require the particular order shown, or sequential order, to achieve desirable results. In certain implementations, multitasking and parallel processing may be advantageous.

## Claims

1. A method comprising:
determining, by one or more processors, an impedance of a sensing element;
determining, by the one or more processors, a capacitance of the sensing element; and
determining, by the one or more processors and based at least in part on (i) the impedance of the sensing element and (ii) the capacitance of the sensing element, (a) a quantity of a first vapor that has reacted with the sensing element and (b) a quantity of a second vapor that has reacted with the sensing element.

2. The method of claim 1, wherein the first vapor comprises an electrolyte vapor and the second vapor comprises a water vapor.

3. The method of claim 1, wherein the sensing element comprises a polymer material and an ionic salt material.

4. The method of claim 1, further comprising:
causing transmission of, by the one or more processors and to the sensing element, a sinusoidal excitation signal having a frequency that is based at least in part on one or more properties of the sensing element, wherein determining the impedance of the sensing element is based at least in part on the sinusoidal excitation signal.

5. The method of claim 1, wherein determining (a) the quantity of the first vapor and (b) the quantity of the second vapor comprises:
solving, by the one or more processors, (i) a first equation that specifies a relationship between the impedance of the sensing element, the quantity of the first vapor, and the quantity of the second vapor, and (ii) a second equation that specifies a relationship between the capacitance of the sensing element, the quantity of the first vapor, and the quantity of the second vapor.

6. The method of claim 1, further comprising:
determining, by the one or more processors, that the quantity of the first vapor satisfies a threshold quantity; and
causing, by the one or more processors, a message to be displayed via a user interface, wherein the message alerts a user that the quantity of the first vapor satisfies the threshold quantity.

7. The method of claim 1, further comprising:
causing, by the one or more processors, a safety protocol to be performed based at least in part on the quantity of the first vapor.

8. The method of claim 7, wherein the safety protocol comprises one or more of (i) venting a battery or (ii) disconnecting the battery.

9. The method of claim 1, wherein the quantity of the first vapor is determined based at least in part on a sinusoidal excitation signal applied to the sensing element.

10. The method of claim 9, further comprising:
causing transmission of, by the one or more processors and to the sensing element, an excitation pulse;
determining a second quantity of the first vapor based at least in part on the excitation pulse; and
determining an error value associated with the second quantity of the first vapor based at least in part on a comparison of the second quantity of the first vapor and the quantity of the first vapor.

11. A system comprising:
a user interface; and
one or more processors in communication with the user interface, the one or more processors configured to:
determine an impedance of a sensing element;
determine a capacitance of the sensing element; and
determine, based at least in part on (i) the impedance of the sensing element and (ii) the capacitance of the sensing element, (a) a quantity of a first vapor that has reacted with the sensing element and (b) a quantity of a second vapor that has reacted with the sensing element.

12. The system of claim 11, wherein the first vapor comprises an electrolyte vapor and the second vapor comprises a water vapor.

13. The system of claim 11, wherein the sensing element comprises a polymer material and an ionic salt material.

14. The system of claim 11, wherein the one or more processors are further configured to:
cause transmission of, to the sensing element, a sinusoidal excitation signal having a frequency that is based at least in part on one or more properties of the sensing element, wherein determining the impedance of the sensing element is based at least in part on the sinusoidal excitation signal.

15. An apparatus comprising:
one or more processors; and
a memory storing instructions that, when executed by the one or more processors, cause the apparatus to:
determine an impedance of a sensing element;
determine a capacitance of the sensing element; and
determine, based at least in part on (i) the impedance of the sensing element and (ii) the capacitance of the sensing element, (a) a quantity of a first vapor that has reacted with the sensing element and (b) a quantity of a second vapor that has reacted with the sensing element.
